# EUROPEAN PATENT APPLICATION

(11) **EP 3 988 061 A1**
(43) Date of publication of application: **27.04.2022**
(21) Application number: 21200224.0
(22) Date of filing: 14.03.2014
(51) Int. Cl.: A61F 2/82, A61F 2/95, A61B 5/01, A61B 5/0205, A61B 5/0215, A61B 5/026, A61B 5/1473, A61B 5/06, A61B 5/029

(54) **STENT MONITORING ASSEMBLY AND METHOD OF USE THEREOF**

(30) Priority: 15.03.2013 US 201361787861 P
(62) Divisional of application: 14762269.0
(71) Applicant: Canary Medical Inc., Vancouver, BC V6E 3R5 (CA)
(72) Inventor: HUNTER, William L., Vancouver, V6E 3R5 (CA)
(74) Representative: Forresters IP LLP

(57) **Abstract**

Assemblies are provided comprising a stent and a sensor positioned on and/or in the stent. Within certain aspects the sensors are wireless sensors, and include for example one or more fluid pressure sensors, contact sensors, position sensors, accelerometers, pulse pressure sensors, blood volume sensors, blood flow sensors, blood chemistry sensors, blood metabolic sensors, mechanical stress sensors and/or temperature sensors. Within certain aspects these stents may be utilized to assist in stent placement, monitor stent function, identify complications of stent treatment, monitor physiologic parameters and/or medically image a body passageway, e.g., a vascular lumen.

## Description

### CROSS-REFERENCE TO RELATED APPLICATION

This application claims the benefit under 35 U.S.C. § 119(e) of U.S. Provisional Patent Application No. 61/787,861 filed March 15, 2013, which application is incorporated herein by reference in its entirety.

### FIELD OF THE INVENTION

The present invention relates generally to the field of vascular and nonvascular stents and, more particularly, to stents for use in monitoring a variety of medical conditions, including, for example, development of restenosis, stent obstruction, and/or other diseases

### BACKGROUND

### Description of the Related Art

Stents are generally cylindrical, flexible, hollow, scaffold-like medical devices that can be inserted into body lumens to physically hold open structures and/or passageways (typically tubular organ structures such as blood vessels, the gastrointestinal tract, the urinary tract, the respiratory tract, or the male and female reproductive tracts) which have become blocked or partially obstructed thereby reducing or eliminating the movement of materials through them. The stent is usually placed percutaneously (e.g. vascular stents) or via insertion through a natural orifice (e.g. the mouth, nose, anus) into the affected organ in a compressed form and then expanded into place (often by inflating a balloon or through the use of "self-expanding" stents) to open the organ lumen back up to its original size and shape. Stents can be utilized to treat and/or prevent a wide variety of diseases and/or conditions resulting from lumen narrowing or obstruction; whether due to an injury or external compression of the vessel wall (a benign or malignant tumour, abscess, cyst), a disease process occurring within the vessel wall (e.g., cancer, atherosclerosis, inflammation, scarring or stenosis), and/or a disease processes occurring on the surface (or in the lumen) of the vessel wall (thrombus, atherosclerosis, retenosis, tumor growth, inflammation and scarring, biliary and urinary "stones", mucous impaction, etc.). Stents can be used in a wide variety of tubular body passageways to preserve the normal movement of luminal materials (blood, digestive contents, digestive enzymes and bile, air, urine, reproductive materials) through them, including for example, vascular structures (e.g., coronary, carotid, cerebral, vertebral, iliac, femoral, popliteal, tibial, mesenteric, pulmonary, and other branches of these arteries; large veins such as the superior and inferior vena cava and veins of the neck, upper and lower extremities), gastrointestinal structures (e.g., esophagus, duodenum, small intestine, colon, biliary tract and pancreatic ducts), pulmonary structures (e.g., to hold open the trachea, bronchi, bronchioles or alveoli), urinary system structures (collecting system, ureters, urethra), female and male reproductive system structures (e.g., to maintain patency of the fallopian tubes, prostatic urethra), sinuses structures in the head and skull (maxillary sinus, frontal sinus, lacrimal duct), and inner ear structures (tympanostomy tubes).

Typically, stents are composed of metallic (stainless steel, titanium, platinum, nitinol, cobalt chromium, etc.) and/or polymeric components (degradable and non-degradable polymers), and are frequently constructed to have either a unitary structure, or composed of multiple components (e.g., bifurcated stent systems). Stents may be non-degradable, partially degradable, or fully degradable. In addition, stents may be coated with one or more different compositions, including both polymers and drugs (see, e.g., U.S. Patent Nos. 8,003,157, 7,294,145, 8,277,867, 8,277,833, as well as U.S Patent Application Nos.US 2005/0181011 and U.S. Patent No. 5,716,981). Representative examples of stents include those disclosed in U.S. Patent Nos. 6,852,153, 7,942,923, 7,753,947, 7,879,082, and 8,287,588.

One of the principle uses of stents is in the treatment of coronary vascular disease, peripheral vascular disease, and cerebral vascular disease. Briefly, coronary vascular disease typically begins with the development of a stenosis, or blockage in the coronary vasculature (right coronary artery, left coronary artery, left anterior descending artery, left circumflex artery, coronary sinus and branches of these); peripheral vascular disease is most often due to stenosis or blockage of the arteries of the leg (common iliac artery, iliac artery, femoral artery, superficial femoral artery, popliteal artery and branches of these), kidneys (renal arteries), or upper limb; and cerebral vascular disease involves arteries of the head and neck (common carotid artery, internal carotid artery and branches of these, cerebral arteries, vertebral artery), although any blood vessel in the body can be so affected. Partial blockage of one or more coronary arteries is often due to the development and progression of arteriosclerotic plaque formation and results in angina (pain and shortness of breath with exercise), while complete blockage of a coronary artery is usually due to plaque rupture and thrombus formation and results in acute coronary syndrome (ACS) and/or myocardial infarction (heart attack). In peripheral arterial disease, partial obstruction of blood vessels in the leg results in claudication (pain or "heaviness" with walking or exercise) and complete vascular obstruction leads to acute ischemia and gangrene; while in cerebral vascular disease narrowing of the blood vessels supplying the brain results in syncope (fainting), dizziness and transient numbness, weakness and speech abnormalities (to name a few symptoms) while complete obstruction results in cerebral vascular accidents (CVA or "strokes" ) in the brain and permanent neurological deficits. In order to address problems caused by either stenosis or obstruction, a stent can be delivered to the site of blockage, typically on a delivery device designed to deliver and deploy the stent, and opened up across the lesion to restore blood flow downstream. A common method of deploying a stent is as follows: a catheter is inserted into the blood stream (often via the femoral artery in the groin) and advanced through the blood stream until it reaches the site of the narrowing or obstruction; it is then advanced across the lesion; the lesion is then opened using a balloon alone (angioplasty) or a stent crimped over an expandable balloon catheter (direct stenting); after deployment and opening of the artery, an expanded stent is then left in place to hold open the lumen of the formerly blocked vessel. In "self-expanding" stents, a balloon is not required to open up the stent, but rather the stent expands in place after deployment from a delivery catheter. Examples of such procedures are described in U.S. Patent No. 5,749,824, WO 98/36709. In nonvascular stent placement, a similar procedure is followed although the stent often gains access to the body via a natural orifice (mouth, nose, anus) and is usually maneuvered into place under direct vision (endoscopy) prior to expansion and deployment.

While there have recently been many advances in the construction, drug-loading, and delivery of stents, there are yet a number of deficiencies that have not yet been addressed.

Accurate placement, deployment, and full expansion of stents continues to be a challenge, particularly in the vasculature, where primarily indirect visualization techniques, such as angiography, are used for stent placement; angiography (radio-opaque dye running through the bloodstream) shows only the vascular luminal anatomy and gives no information about the vessel wall anatomy (which is often the critical diseased segment being treated). Full and complete deployment (full opening) of the stent is often difficult to confirm with angiography alone. Long lesions and branched lesions (disease occurring at bifurcation points in the artery) often require the use of multiple stents, overlapping stents or bifurcated stents; accurate placement and determining the amount of overlap (greater overlap between continuous or connected stents increases the risk of ultimate failure) between adjacent stents is also difficult to confirm. Stents containing sensors capable of providing the physician with real-time information about the vascular wall anatomy, balloon and vessel wall pressure, stent location within the vessel wall, full expansion and deployment of the stent, patency/luminal size within the stent, contact and overlap between adjacent/connected stents, blood flow rates through the device, and post deployment placement confirmation would be greatly beneficial to the attending physician and significantly lower long-term complication rates.

After deployment, monitoring the development of potential complications (kinking, stent fracture, restenosis, thrombosis, malaposition) would assist in better managing the patient post-operatively and alert both the patient and the doctor to the development of potentially serious side effects. Also, monitoring the surface characteristics of the stent to determine healing of the device within the artery (coverage of the luminal stent surface with endothelium), can help determine when and if the patient can be removed from their anti-platelet (or anti-coagulant) therapy. Ongoing monitoring of physiological parameters such as, pulse rate, pulse pressure, blood pressure and blood flow rates can provide useful information about systemic and regional cardiovascular function in general. Additionally, in the case of biodegradable and bioerodible stents, sensors embedded on the surfaces (luminal and adluminal) and at varying depths within the (typically) polymeric stent can provide useful information as to the dissolution rate and ultimate complete bioabsorption of the stent. In drug eluting stents, sensors can be used to monitor the release of therapeutic agents from the device.

Post-operative, in-hospital monitoring of patients receiving stents is conducted through personal visits by the hospital staff and medical team, physical examination of the patient, medical monitoring (vital signs, telemetry, etc.), and diagnostic imaging studies and blood work as required. Once the patient is discharged from hospital, stent performance and patient progress is checked during periodic doctor's office visits where a thorough history, physical exam and supplemental imaging and diagnostic studies are used to monitor patient progress and identify the development of any potential complications. During such visits, the clinician typically evaluates physical signs and symptoms, conducts studies as indicated (ECG, echocardiography, angiography), and questions the patient to determine activity levels, daily functioning, pain, and rehabilitation progress.

Unfortunately, most of the patient's recuperative period occurs between hospital and office visits. It can, therefore, be very difficult to accurately measure and follow the development or worsening of symptoms and correlate "real life" stent performance with patient activity levels, exercise tolerance, rehabilitation programs and medications. For much of this information, the physician is dependent upon subjective patient self-reporting to obtain insight into post-operative treatment effectiveness, recovery and rehabilitation progress; in many cases this is further complicated by a patient who is uncertain what to look for, has no knowledge of what "normal/expected" post-operative recovery should be, is non-compliant, or is unable to effectively communicate their symptoms. Furthermore, identifying and tracking complications (in and out of hospital) prior to them becoming symptomatic, arising between doctor visits, or those whose presence is difficult (or impossible) to detect would also provide beneficial, additional information to the management of stent patients. At present, neither the physician nor the patient has access to the type of "real time," continuous, objective, stent performance measurements that they might otherwise like to have. Being able to monitor *in situ* stent function can provide the physician with valuable objective information during office visits; furthermore, the patient can take additional readings at home at various times (e.g. when experiencing pain, during exercise, after taking medications, etc.) to provide important complementary clinical information to the doctor (which can be sent to the healthcare provider electronically even from remote locations) and can provide the patient with either an early warning indicator to seek assistance or to provide them with reassurance.

The present invention discloses novel stents which overcome many of the difficulties of previous stents, methods for constructing and utilizing these novel stents, and further provides other related advantages.

### SUMMARY

Briefly stated, assemblies are provided comprising a stent and a sensor to monitor among other things, the anatomy (and general well-being) of the tissues surrounding the stent, the integrity or efficaciousness of the stent, the complete opening and accurate deployment of the stent, the relationship of the stent to other stents or stent segments, a disease process, the movement of body fluids through the stent, healing of the stent within the body, the failure or impending failure of the stent due to a disease or other process (e.g., restenosis, inflammation, benign or malignant tumor growth, clot formation), injury, or an interventional procedure (e.g., surgery). Representative stents suitable for use within the present invention include, for example, vascular (e.g., coronary artery, carotid artery, cerebral artery, vertebral artery, renal artery, iliac artery, mesenteric artery and arteries of the upper and lower extremities as well as branches of all the aforementioned aterial vessels; venous stents), gastrointestinal (e.g., esophageal, biliary, duodenal, colonic, and pancreatic), pulmonary (e.g., to hold open trachea, bronchi, brochioles or alveoli), head and neck (sinus, lacrimal, tympanic) and genitourinary (e.g.,, ureteral, urethral, fallopian tube, prostate) stents.

Within one aspect of the present invention assemblies are provided comprising a stent and a sensor positioned on or within said stent. Such stents may be positioned within a wide variety of lumens, including for example naturally occurring body passageways (e.g., vasculature such as coronary, carotid, cerebral, and vertebral vessels, as well as renal, iliac and arteries of the lower extremities; pulmonary airways (e.g., trachea, bronchi and other air passages within the lungs, including the bronchioles or alveoli), gastrointestinal structures (e.g., esophagus, duodenum, colon, anus, biliary ducts and pancreatic ducts ), head and neck (sinuses, lacrimal duct, typanostomy tubes), and genitourinary (e.g., ureteral and urethral, fallopian tube, prostate), surgically created body passageways (cerebral shunts, spinal shunts, pulmonary shunts, hepatic shunts, ileostomies, colostomies, surgical drains, tympanostomy tubes), and passageways created or caused by injury or a disease process.

Within various embodiments the assembly comprises a stent and one or more sensors positioned on or within the stent, including for example, one or more sensors positioned on the outer wall of the stent, the inner wall of the stent, and or within the stent material itself. Within related embodiments, the one or more sensors may be placed on the luminal surface, adluminal surface and or implanted with or contained within the stent itself.

A wide variety of sensors can be utilized within the present invention, including for example, fluid pressure sensors, contact sensors, position sensors, accelerometers, vibration sensors, pulse pressure sensors, blood volume sensors, blood flow sensors, blood chemistry sensors, blood metabolic sensors, mechanical stress sensors, and temperature sensors. Within one embodiment the sensor can be connected with other medical devices that can be utilized to delivery one or more drugs. Within other embodiments the one or more sensors can be a wireless sensor, and / or a sensor that is connected to a wireless microprocessor.

Within particularly preferred embodiments a plurality of sensors are positioned on the stent, and within yet other embodiments more than one type of sensor is positioned on the stent. Within other related embodiments the plurality of sensors are positioned on or within the stent at a density of greater than 1, 2, 3, 4, 5, 6, 7. 8. 9. 10 or 20 sensors per square centimeter. Within other embodiments the plurality of sensors are positioned on or within the stent at a density of greater than 1, 2, 3, 4, 5, 6, 7, 8, 9. 10 or 20 sensors per cubic centimeter. Within either of these embodiments there can be less than 50, 75, 100, or 200 sensors per square centimeter, or per cubic centimeter.

Within other embodiments of the invention each assembly has a unique device identification number. Within further embodiments one or more (or each) of the sensors have a unique sensor identification number. Within yet other embodiments one or more (or each) of the sensors is uniquely defined within a specific position on or within the stent.

Within yet other aspects of the invention, assemblies are provided comprising a stent and one or more of the sensors provided herein, wherein the sensor measures or detects one or more measurements of cardiac function, including for example, cardiac output, stroke volume, ejection fraction, systolic blood pressure, diastolic blood pressure, mean arterial pressure, systemic vascular resistance, total peripheral resistance, temperature, and / or the development of restenosis, clotting, or partial or complete obstruction of luminal fluid flow within a subject. Within other aspects of the invention, assemblies are provided comprising a stent and one or more of the sensors provided herein, wherein the sensor measures or detects surface (luminal) contact and is able to measure healing (in vascular stents this is typically endothelialization) and the degree/extent of coverage of the luminal surface of the stent with biological tissue; such information can be used by the clinician to determine if the patient remains at risk for thrombosis and whether or not anti-coagulation therapy needs to be continued.

Within certain embodiments of the invention, the stent is a drug-eluting stent which can be, optionally, coated with or containing one or more polymers.

Within other aspects of the invention use of the assemblies described herein are provided for the measurement of a cardiac function (as described herein), and/or to medically image and/or self-diagnose one or more aspects of cardiac function, disease, stent integrity and/or stent efficaciousness.

Within further aspects of the present invention methods are provided for monitoring a stent comprising (a) transmitting a wireless electrical signal from a location outside the body to a location inside the body; b) receiving the signal at a sensor positioned on a stent located inside the body, c) powering the sensor using the received signal, d) sensing data at the sensor, and e) outputting the sensed data from the sensor to a receiving unit located outside of the body. Within various embodiments the stent may comprise any of the assemblies provided herein.

Within other aspects, non-transitory computer-readable storage medium whose stored contents configure a computing system to perform a method are provided, comprising: a) identifying a subject, the identified subject having at least one wireless stent, each wireless stent having one or more wireless sensors, b) directing a wireless interrogation unit to collect sensor data from at least one of the respective one or more wireless sensors, and c) receiving the collected sensor data. Within certain embodiments, such methods may optionally further comprise the steps of a) identifying a plurality of subjects, each identified subject having at least one wireless stent, and each wireless stent having one or more wireless sensors, b) directing a wireless interrogation unit associate with each identified subject to collect sensor data from at least one of the respective one or more wireless sensors, c) receiving the collected sensor data, and d) aggregating the collected sensor data. Within yet further embodiments, such methods may optionally further comprise the steps of a) removing sensitive subject data from the collected sensor data, and b) parsing the aggregated data according to a type of sensor. Within related embodiments the stored contents configure a computing system to perform a method, wherein direct the wireless interrogation unit include directing a control unit associated with the wireless interrogation unit. Any of the assemblies, stents, and/or sensors described herein may be utilized within such methods.

Within another aspect of the invention methods for determining degradation of a stent are provided, comprising the steps of a) providing to a body passageway of a subject an assembly comprising a stent and one or more sensors positioned on the surface and/or at varying depths within the biodegradable/bioerodible stent, and b) detecting a change in a sensor, and thus determining degradation rate and/or complete degradation of the stent. Within various embodiments said sensor is capable of detecting one or more physiological (e.g., contact, fluid flow, pressure and/or temperature) and/or locational (e.g., location within the subject) parameters. Within further embodiments the step of detecting is a series of detections over time, and optionally, the method may further comprise the step of determining the rate of degradation of the stent, and/or estimating the time for complete degradation of the stent.

Within yet other aspects of the invention methods are provided for imaging a stent, or an assembly comprising a stent with sensors, comprising the steps of detecting the changes in sensors in, on, and or within a stent over time, and wherein the stent comprises sensors at a density of greater than 1, 2, 3, 4, 5, 6, 7, 8, 9. 10 or 20 sensors per square centimeter. Within other aspects the stent comprises sensors at a density of greater than 1, 2, 3, 4, 5, 6, 7, 8, 9, 10 or 20 sensors per cubic centimeter. Within either of these embodiments there can be less than 50, 75, 100, or 200 sensors per square centimeter, or per cubic centimeter.

As noted above, a wide variety of sensors can be utilized therein, including for example, fluid pressure sensors, contact sensors, position sensors, accelerometers, vibration sensors, pressure sensors, blood volume sensors, blood flow sensors, blood chemistry sensors, blood metabolic sensors, mechanical stress sensors, and temperature sensors. Within various embodiments the stent can be a vascular, gastrointestinal, pulmonary, sinus, or genitourinary stent, and optionally, can be biodegradable, partially biodegradable, or non-biodegradable. Within yet other embodiments, the sensor is a wireless sensor, and / or a sensor connected to a wireless microprocessor. By imaging the stent in this manner, the integrity of the stent can be wirelessly interrogated and the results reported on a regular basis. This permits the health of the subject to be checked on a regular basis or at any time as desired by the subject and/or physician.

The details of one or more embodiments are set forth in the description below. Other features, objects and advantages will be apparent from the description, the drawings, and the claims. In addition, the disclosures of all patents and patent applications referenced herein are incorporated by reference in their entirety.

### BRIEF DESCRIPTION OF THE DRAWINGS

Figure 1 is an illustration of one representative stent with sensors positioned therein (including for example, blood flow sensors, pressure sensors, position sensors or location markers and/or pH sensors).
Figure 2 is an illustration of one representative stent with sensors positioned therein, showing blood movement through the stent.
Figure 3A is an illustration of one representative stent with a variety of openings within the stent struts. Figure 3B depicts the placement of one or more sensors within one of the openings of the strut.

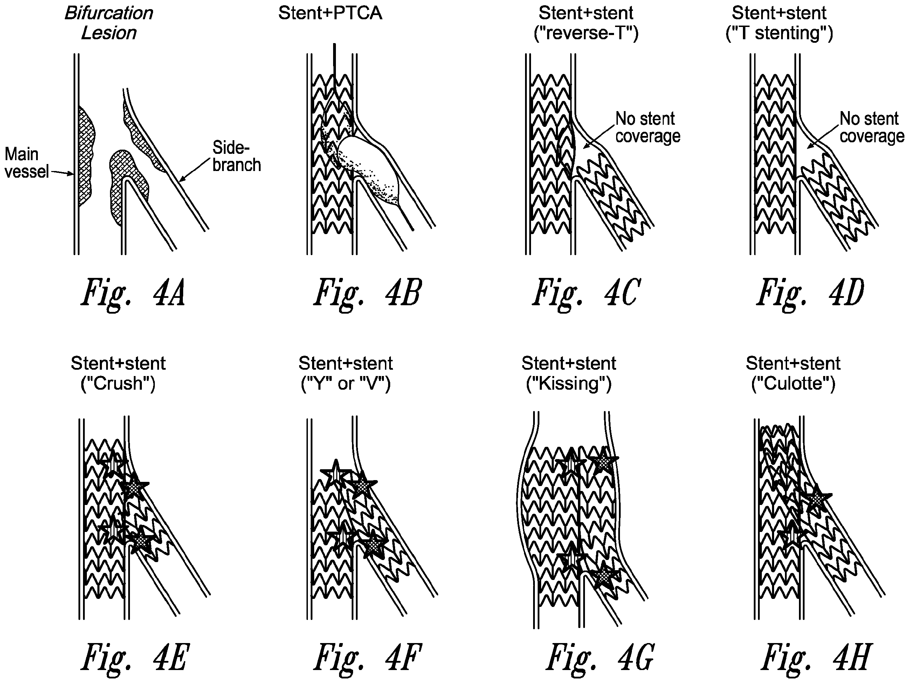

Figure 4 is a schematic of various types of stent placement, and contact sensors which can aid in this placement. Figure 4A illustrates a site of bifurcation with stenosis occurring at multiple points in the vessel. Figure 4B illustrates a stent with PTCA. Figure 4C illustrates a stent plus stent deployment (also referred to as a "reverse-T"). Figure 4D illustrates a stent plus stent deployment (referred to as "T stenting"). Figure 4E illustrates a stent plus stent deployment referred to as a "Crush". Figure 4F illustrates a stent plus stent deployment referred to as a "Y" or "V". Figure 4G illustrates a stent plus stent deployment referred to as "Kissing". Figure 4H illustrates a stent plus stent deployment referred to as a "Culotte".
Figure 5 is a schematic illustration of contact sensors that can be utilized to aid and or assist the placement of overlapping stents.
Figure 6 illustrates the medical imaging of vascular anatomy through sensors which can detect positional movement.
Figure 7 illustrates the medical imaging of vasculature by sensors which can detect positional movement due to vascular pathology.
Figure 8 illustrates an information and communication technology system embodiment arranged to process sensor data.
Figure 9 is a block diagram of a sensor, interrogation module, and control unit according to one embodiment of the invention.
Figure 10 is a schematic illustration of one or more sensors positioned on a stent within a subject which is being probed for data and outputting data, according to the disclosure herein.

### DETAILED DESCRIPTION OF THE INVENTION

As noted above, stents are provided with a number of sensors to monitor the accurate placement and deployment of the stent(s) in the body, the anatomy and pathology of the tissue surrounding the stent, integrity and efficaciousness of the stent, normal and abnormal healing of the tissues in contact with the stent, function of the tissues and organ systems in contact with the stent, degradation and dissolution of the stent (in the case of degradable stents), as well as to monitor the failure or impending failure of the stent due to a disease or other process (e.g., restenosis, thrombosis, inflammation, benign or malignant tumor growth). Prior to setting forth the invention however, it may be helpful to an understanding thereof to first set forth definitions of certain terms that are used hereinafter.

"Stent" refers to a medical device that can be utilized to hold open body structures and/or passages, and can be utilized to treat and/or prevent a wide variety of diseases and/or conditions resulting from lumen narrowing or obstruction; whether due to an injury or external compression of the vessel wall (a benign or malignant tumour, abscess, cyst), a disease process occurring within the vessel wall (e.g., cancer, atherosclerosis, inflammation, scarring or stenosis), and/or a disease processes occurring on the surface (or in the lumen) of the vessel wall (thrombus, atherosclerosis, retenosis, tumor growth, inflammation and scarring, biliary and urinary "stones", mucous impaction, etc.), and/or an operation or other medical intervention.

Stents can be used in a wide variety of variety of tubular body passageways to preserve the normal movement of luminal materials (blood, digestive contents, digestive enzymes and bile, air, urine, reproductive materials) through them, including for example, vascular structures (e.g., coronary, carotid, cerebral, vertebral, iliac, femoral, popliteal, tibial, mesenteric, pulmonary, and other branches of these arteries; large veins such as the superior and inferior vena cava and veins of the neck, upper and lower extremities), gastrointestinal structures (e.g., esophagus, duodenum, small intestine, colon, biliary tract and pancreatic ducts), pulmonary structures (e.g., to hold open the trachea, bronchi, bronchioles or alveoli), urinary system structures (collecting system, ureters, urethra), female and male reproductive system structures (e.g., to maintain patency of the fallopian tubes, prostatic urethra), sinuses structures in the head and skull (maxillary sinus, frontal sinus, lacrimal duct), and inner ear structures (tympanostomy tubes).

Typically, stents are composed of metallic or polymeric components, and have a unitary structure, or multiple components (e.g., a bifurcated stent system). Stents may be non-degradable, partially degradable, or fully degradable. In addition, stents may be coated with one or more different compositions, including both polymers and drugs (including biologics and stem cells). Representative examples of stents include those disclosed in U.S. Patent Nos. 6,852,153, 7,942,923, 7,753,947, 7,879,082, and 8,287,588, as well as various publications (see, e.g., "Open Stent Design: Design and analysis of self expanding cardiovascular stents", by Craig S. Bonsignore, CreateSpace Independent Publishing Platform, Nov. 2012, and "Coronary Stents" by Sigwart and Frank (eds.), Springer, 2012)

Within preferred embodiments the stents of the present invention have a Unique Device Identification ("UDI") number, and each of the sensors on the stent have a Unique Sensor Identification ("USI").

"Sensor" refers to a device that can be utilized to measure one or more different aspects of a body, of a stent inserted within a body, and/or the integrity, impact, efficaciousness or effect of the stent inserted within a body. Representative examples of sensors suitable for use within the present invention include, for example, fluid pressure sensors, contact sensors, position sensors, pulse pressure sensors, blood volume sensors, blood flow sensors, chemistry sensors (e.g., for blood and/or other fluids), metabolic sensors (e.g., for blood and/or other fluids), accelerometers, mechanical stress sensors and temperature sensors. Within certain embodiments the sensor can be a wireless sensor, or, within other embodiments, a sensor connected to a wireless microprocessor. Within further embodiments one or more (including all) of the sensors can have a Unique Sensor Identification number ("USI") which specifically identifies the sensor.

A wide variety of sensors (also referred to as Microelectromechanical Systems or "MEMS", or Nanoelectromechanical Systems or "NEMS", and BioMEMS or BioNEMS, see generally https://en.wikipedia.org/wiki/MEMS) can be utilized within the present invention. Representative patents and patent applications include U.S. Patent No. 7,383,071 and U.S. Publication No. 2010/0285082. Representative publications include "Introduction to BioMEMS" by Albert Foch, CRC Press, 2013; "From MEMS to Bio-MEMS and Bio-NEMS: Manufacturing Techniques and Applications by Marc J. Madou, CRC Press 2011; "Bio-MEMS: Science and Engineering Perspectives, by Simona Badilescu, CRC Press 2011; "Fundamentals of BioMEMS and Medical Microdevices" by Steven S. Saliterman, SPIE-The International Society of Optical Engineering, 2006; "Bio-MEMS: Technologies and Applications", edited by Wanjun Wang and Steven A. Soper, CRC Press, 2012; and "Inertial MEMS: Principles and Practice" by Volker Kempe, Cambridge University Press, 2011; Polla, D. L., et al., "Microdevices in Medicine," Ann. Rev. Biomed. Eng. 2000, 02:551-576; Yun, K. S., et al., "A Surface-Tension Driven Micropump for Low-voltage and Low-Power Operations," J. Microelectromechanical Sys., 11:5, October 2002, 454-461; Yeh, R., et al., "Single Mask, Large Force, and Large Displacement Electrostatic Linear Inchworm Motors," J. Microelectromechanical Sys., 11:4, August 2002, 330-336; and Loh, N. C., et al., "Sub-10 cm3 Interferometric Accelerometer with Nano-g Resolution," J. Microelectromechanical Sys., 11:3, June 2002, 182-187; all of the above of which are incorporated by reference in their entirety.

In order to further understand the various aspects of the invention provided herein, the following sections are provided below: A. Stents and Their Use; B. Stents with Sensors Located Within the Stent; C. Stent Placement, Deployment and Connections; D. Partially or Fully Biodegradable Stents; E. Stent Coatings; F. Drug-Eluting Stents; G. Methods for Monitoring Infection in Stents; H. Further Uses of Sensor-containing Stents in Healthcare; I. Generation of Power from Stents; J. Medical Imaging and Self-Diagnosis of Assemblies Comprising Stents, Predictive Analysis and Predictive Maintenance; K. Methods of Monitoring Assemblies Comprising Stents; and L. Collection, Transmission, Analysis, and Distribution of Data from Assemblies Comprising Stents.

### A. STENTS AND THEIR USE

As noted above, stents are used to open up and maintain the lumen of a diseased body passageway (e.g. artery, gastrointestinal tract, urinary tract), but have found their greatest utility in the vasculature. Briefly, a stent is inserted into body a lumen to physically hold open structures and/or passageways (typically tubular organ structures such as blood vessels, the gastrointestinal tract, the urinary tract, the sinuses of the skull, the respiratory tract, or the male and female reproductive tracts) which have become blocked or partially obstructed thereby reducing or eliminating the movement (typically fluids, solids or air) through them. The stent is usually placed percutaneously (e.g. vascular stents are often inserted into the vasculature via the femoral artery in the groin and then maneuvered through the blood stream under radiological guidance until they reach the diseased blood vessel) or via insertion through a natural orifice (e.g. the mouth, nose, anus) and placed under direct vision (endoscopy) into the affected organ. Most often the stent is delivered to the deployment site in a compressed form and then expanded into place (often by inflating a balloon or through the use of "self-expanding" stents) to open the organ lumen back up to its original size and shape. The symptoms of blockage or obstruction (e.g. chest pain, claudication, neurological deficit, dysphagia, bowel obstruction, jaundice, difficulty breathing, infertility, urinary obstruction, sinus pain) depend upon the organ affected and restoration of normal anatomy and lumen function is the goal of stent treatment. Stent failure can be due to a multitude of causes but includes things such improper placement, improper sizing, incomplete opening or deployment, tissue ingrowth into the stent lumen (restenosis, tumor cell growth, inflammation), luminal obstruction (clot, biliary stone, kidney stone), stent fracture, stent kinking and stent migration. Stents containing sensors able to assist the physician in their proper placement and deployment, and stents capable of ongoing monitoring to detect evidence of partial and/or complete obstruction, would have significant benefits over existing devices.

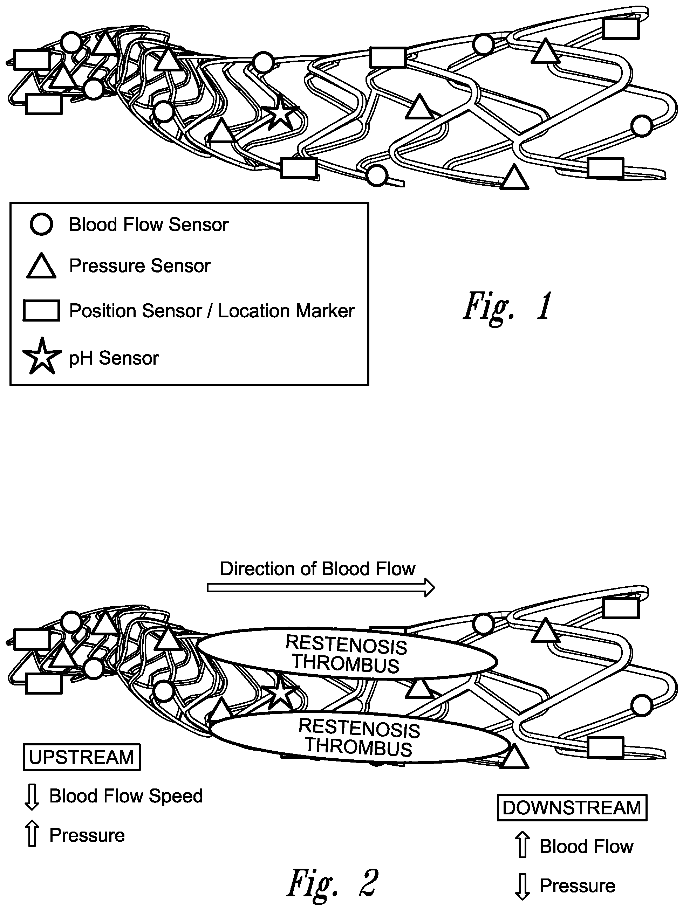

Figure 1 is an illustration of one representative stent with sensors positioned therein. Figure 2 is an illustration wherein some of the sensors are positioned in a location exposed to the blood flowing through the stent. A wide variety of sensors can be placed on the inner (luminal) wall of the stent, within the stent, and/or, on the outer (adluminal) wall of the stent. Representative sensors that can be utilized within a stent include fluid pressure sensors, contact sensors, position sensors, pulse pressure sensors, blood volume sensors, blood flow sensors, blood chemistry sensors, blood (and tissue) metabolic sensors, accelerometers, mechanical stress sensors, vibration sensors and temperature sensors.

Within various embodiments vascular stents (coronary, peripheral and cerebral) of the present invention can have a variety of sensors capable of detecting and differentiating types of normal vascular healing versus stenosis, restenosis, and/or thrombosis. Blood flow, fluid pressure and blood volume sensors located on the luminal surface are able to detect the presence and location of a stenosis due to the increased blood flow speed and increased blood (and pulse) pressure at the site of a stenosis (relative to normal pressures). Stenosis due to neointimal hyperplasia or clot formation can be detected as "dead spots" and/or altered readings on the luminal surface as blood flow sensors, blood metabolic and/or blood chemistry sensors become covered by vascular tissue or clot; while adluminal pressure sensors and accelerometers will not show changes in adluminal pressure or stent wall deformation. Metabolic sensors and chemistry sensors are capable of determining the difference between stenosis (normal pH and physiologic readings) and clot (lowered pH and altered physiologic readings). Lastly, complete coverage of the luminal surface of the stent in the absence of altered pressure, blood flow rates, stent deformation and metabolic/chemistry readings is suggestive of normal healing; that the stent has become endothelialized (covered with the cells that line the body's blood vessels). This indicator of healthy and complete incorporation of the stent within the blood vessel wall (i.e. the stent is no longer exposed to the elements of the bloodstream) has an important clinical consequence - it alerts the clinician that it may be possible to discontinue the patient's (costly and dangerous) anticoagulant therapy since the risk of subacute and delayed thrombosis is now markedly reduced. In the case of biodegradable stents, complete coverage of the luminal surface of the stent and incorporation of it into the vessel wall means that dissolution of the stent is now safe (i.e. stent fragments will not be released into the blood stream).

In addition, subjects requiring stents often have extensive cardiovascular disease resulting in impaired cardiac and systemic circulatory function. For example, subjects receiving stents are at an increased risk for myocardial infarction (heart attack), cerebral vascular accidents (stroke), congestive heart failure, renal failure and arrhythmias. The coronary arteries are critical to the functioning of the heart, and hence, monitoring certain hemodynamic and metabolic parameters within these arteries can provide the clinician with very important information regarding the subject's cardiac, renal and circulatory function. Coronary stents of the present invention can contain fluid pressure sensors, contact sensors, position sensors, pulse pressure sensors, blood volume sensors, blood flow sensors, blood chemistry sensors, blood metabolic sensors, accelerometers, mechanical stress sensors, temperature sensors, and the like, suitable for such purposes. Representative stents of the present invention can be utilized by one of ordinary skill in the art to calculate and monitor important physiologic parameters such as cardiac output (CO), stroke volume (SV), ejection fraction (EV), systolic blood pressure (sBP), diastolic blood pressure (dBP), mean arterial pressure (mAP), systemic vascular resistance (SVR), total peripheral resistance (TPV) and pulse pressure (PP). For example, the FloTrac/Vigileo (Edwards Life Sciences, Irvine, CA) uses pulse contour analysis to calculate stroke volume (SV) and systemic vascular resistance (SVR); the pressure recording analytical method (PRAM) is used by Most Care (Vytech, Padora, Italy) to estimate cardiac output (CO) from analysis of the arterial pressure wave profile. Changes in cardiac output (CO), stroke volume (SV) and ejection fraction (EF) and cardiac index (CI) can be an important in detecting complications such myocardial ischemia and infarction; they can also assist the clinician in implementation and adjusting cardiac medications and dosages. Pulse pressure sensors, pulse contour sensors and heart rate sensors contained on and within stents of the present invention can assist in the detection and monitoring of cardiac arrhythmias and heart rate abnormalities; they too can be used to monitor the subject's response to cardiac medications that effect heart rate and rhythm. Systolic blood pressure (sBP), diastolic blood pressure (dBP), mean arterial pressure (mAP), systemic vascular resistance (SVR) and total peripheral resistance (TPV) readings can be used by the clinician to monitor the dosage and effect of blood pressure lowering medications and pressor (blood pressure increasing) agents. It is obvious that peripheral and cerebral vascular stents implanted in other arteries (renal, iliac, femoral, carotid, etc.) are capable of monitoring virtually all of the above parameters as well.

Vascular stents of the present invention can contain circulatory sensors (as described herein) as well as blood chemistry sensors and blood metabolic sensors suitable for monitoring kidney function. Examples of blood chemistry and metabolic sensors of utility for this embodiment include, but are not limited to, Blood Urea Nitrogen (BUN), Creatinine (Cr) and Electrolytes (Calcium, Potassium, Phosphate, Sodium, etc.). Furthermore, combining metabolic data with hemodynamic data and urinalysis can allow the clinician to calculate the Glomerular Filtration Rate (GFR) which is a very useful measure of kidney function. This information would be of particular utility in the management of dialysis subjects to monitor the timing, effectiveness, and frequency of dialysis therapy.

Within one embodiment of the invention the stent may also comprise one or more temperature sensors. These sensors may be utilized to track both the discrete temperature of the blood, vessel wall and surrounding environment, but the change of temperature overtime. Such change in temperature may be utilize to diagnose a possible developing infection (or other disease or condition), and allow a physician or care-giver to treat the infection (or other disease or condition) prior to a full onset

### B. STENTS WITH SENSORS LOCATED WITHIN THE STENT

As noted above, within various aspects of the invention sensors as described herein can be contained within the stent, including for example, within holes in the struts of the stent, or within the struts themselves. As utilized herein, "holes" should be understood to include openings that run entirely through a stent, as well as cavities, depressions, wells, or other openings or partial openings which permit insertion of a sensor within the stent. Representative examples of stents include those described within U.S. Patent Nos. 7,208.010, and 7,179,289.

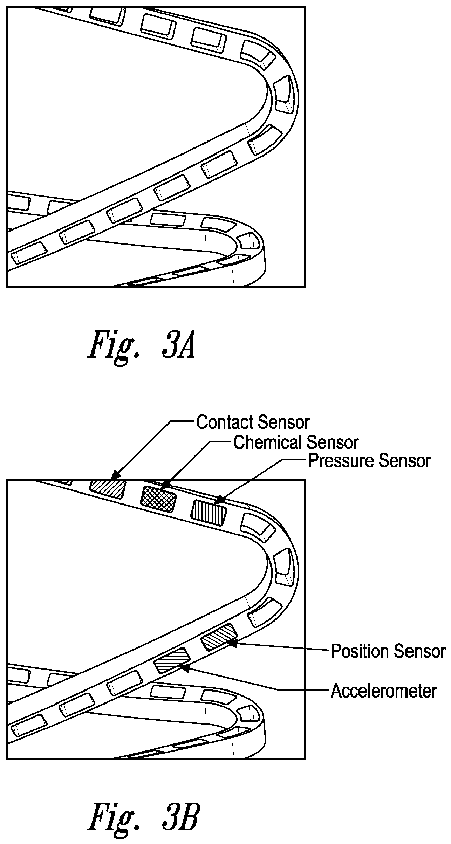

For example, as shown in Figure 3A, one representative stent is provided with a variety of holes within the stent struts. Figure 3B depicts the placement of one or more sensors within one of the openings of the strut.

### C. STENT PLACEMENT, DEPLOYMENT AND CONNECTIONS

Stents of the present invention, within certain embodiments, can provide sensing information to serve a variety of important clinical functions. It is widely accepted that the greater the amount of trauma experienced by the vessel wall during stent placement and deployment, the higher the probability that the stent will ultimately become obstructed (often due to restenosis). Causes of vessel trauma during placement include inaccurate sizing (stents too large for the vessel), difficult placement and deployment (requiring extensive manipulation to place the stent), long lesions, overlapping stents, over-inflation of the balloon or over-expansion of the stent, complicated lesions (including stenting at branch points) and placing stents in tortuous vessels. Accurate placement, sizing, deployment, and full expansion of stents continues to be a challenge, particularly in the vasculature, where primarily indirect visualization techniques, such as angiography, are used for stent placement; angiography (radio-opaque dye running through the bloodstream) shows only the vascular luminal anatomy and gives no information about the vessel wall anatomy (which is often the critical diseased segment being treated) and only limited information about the stent. "Real Time" sensing information from the stent itself is useful to the clinician during placement of the stent to determine: if it is correctly implanted anatomically, if the stent is appropriately sized for the vessel in which it is placed, if it is completely opened (deployed) during balloon expansion (or during self-expansion), if it exerts too much (or too little) pressure against the vessel wall, if stent segments are correctly assembled, if there is an optimal amount of overlap between adjacent stents, if there is kinking or deformation of the stent, if there is cracking or fracturing of the stent, if there is uniform flow through the device - to name but a few important functions. Stents of the present invention can allow the operating physician to monitor many valuable parameters that can lead to better and less traumatic stent placement and deployment.

Improper sizing of the stent relative to the vessel wall in which it is placed can significantly increase the risk of failure (particularly due to restenosis); stents with sensors able to detect the amount, presence and/or absence of pressure and contact with the vessel wall can assist in matching the stent size and degree of expansion (deployment) to that of the vessel wall. Incomplete opening of all, or parts of the stent (known as "incomplete malaposition" - areas where the stent is not in full contact with the vessel wall and projects into the arterial lumen), increases the risk of subsequent clotting (thrombosis) and stent failure; position sensors, contact sensors and accelerometers on the stent can be used to identify and correct areas of incomplete opening (deployment) during stent insertion; "locking" into the fully opened position can be confirmed by sensors on and within the device. Improper positioning (malpositioning) of the stent, either at the time of placement or due to subsequent movement/migration, is also a common complication of stent therapy. Sensor-containing stents of the present invention can be used to confirm proper initial placement and any ensuing migration or relocation within the vessel. Movement of the stent as a whole, or detachment of individual stent segments from each other is another problematic complication of stent insertion and ongoing therapy. Stents of the present invention have the ability to detect movement/detachment of the entire stent, as well as movement and/or detachment of individual segments (or fragments), providing the clinician and patient with valuable diagnostic information. Kinking of the stent during deployment and/or as the result of subsequent movement after placement is also a significant clinical problem if it develops. Stents of the present invention have position sensors and accelerometers distributed throughout the stent capable of detecting deformation and kinking of the stent. Stent cracking and fracture can be a problem with all stents, but particularly in peripheral stents of the lower limb (due to movement of the limb or bending of the stent across the knee joint) and in polymeric degradable stents. Vibration sensors, position sensors, location sensors and accelerometers located throughout the device could alert the clinician and the patient to the development of this complication prior to it developing into an acute emergency.

Within various aspects of the invention assemblies are provided wherein a stent may be composed of a unitary component which is combined with another stent, or of multiple components which need to be placed in the appropriate configuration to ensure proper utility. When the patient has arterial disease and vessel narrowing at branching points in the vascular tree, it is often necessary to use stents (or stent components) than can be placed together *in situ* to match the anatomy of the obstructed segment. For example, Figure 4 is a schematic illustration of various types of multiple stent placement, wherein contact sensors can be utilized to ensure proper placement of the stent. Figure 4A illustrates a site of bifurcation with stenosis occurring at multiple points in the vessel. Figure 4B illustrates a stent with PTCA. Figure 4C illustrates a stent plus stent deployment (also referred to as a "reverse-T"). Figure 4D illustrates a stent plus stent deployment (referred to as "T stenting"). Figure 4E illustrates a stent plus stent deployment referred to as a "Crush". Figure 4F illustrates a stent plus stent deployment referred to as a "Y" or "V". Figure 4G illustrates a stent plus stent deployment referred to as "Kissing". Figure 4H illustrates a stent plus stent deployment referred to as a "Culotte". In each case, (potentially "matched" or complimentary) contact sensors can be used to confirm accurate assembly; accelerometers can be used to confirm anatomical location and conformation; position sensors can monitor movement; flow sensors can confirm vascular patency; and pressure/vessel wall sensors can confirm full deployment and accurate vessel sizing. Taken collectively, this sensing information can create a 3-dimensional image of the vascular and stent anatomy and greatly improve the data available from angiography alone. This dramatically increases the chances of accurate, safe and effective deployment of multiple stents in complicated vascular lesions.

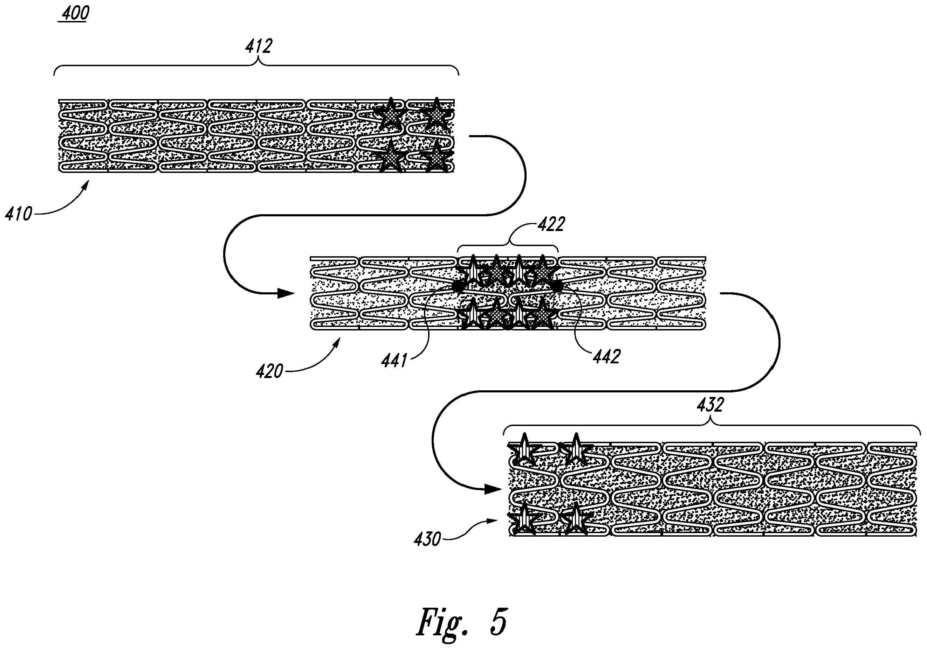

Figure 5 is a schematic illustration of contact sensors that can be utilized to aid and or assist the placement of overlapping stents. Overlapping stents are used in the treatment of long lesions or tortuous lesions where a single stent is insufficient to span the entire length of the diseased segments. While often effective, overlapping stents are more prone to failure and the rate of failure is directly proportional to the degree of overlap between adjacent stents; too much overlap increases failure risk, while too little - particularly if there is a gap between the two stents - is equally problematic. Contact sensors between stents can be used to confirm both the presence and the extent of overlap between adjoining stents. In a preferred embodiment the contact sensors between stents are "matched," or complementary, confirming when the ideal amount of overlap has been achieved between neighboring stents. Furthermore, pressure sensors, position sensors and accelerometers can be used to confirm that the overlapping segments are equally deployed to ensure that there is not a "mismatch" in lumen size in the two stents where they overlap.

### D. PARTIALLY OR FULLY BIODEGRADABLE STENTS

As noted above, stents of the present invention (including for example, vascular (e.g., coronary, carotid, cerebral, vertebral, iliac, femoral and arteries of the lower extremities), gastrointestinal (e.g., esophageal, duodenal, colonic, biliary and pancreatic), pulmonary (e.g., to hold open the trachea, bronchus, bronchi or alveoli), head and neck (sinus, lacrimal, tympanostomy), and genitourinary (e.g., ureteral and urethral, prostate, fallopian tube) may be comprised of one or more biodegradable polymers. Such stents may be fully, or partially biodegradable and or resorbable. Representative examples of such stents include for example U.S. Patent App. Nos. 2009/0192588, 2007/0270940, and 2003/0104030, and U.S. Patent Nos. 6,387,124, 6,869,443 and 7,044,981.

Placement of sensors as described herein on or within a biodegradable or partially biodegradable stent (at varying depths within the polymer) allows a determination of degradation of the stent, as well as, optionally, the rate of biodegradation or resorption of the stent. Hence, within one aspect of the invention methods are provided for determining degradation of a stent are provided, comprising the steps of a) providing to a body passageway of a subject an assembly comprising a stent and one or more sensors, and b) detecting a change in a sensor, and thus determining degradation of the stent. Within various embodiments the sensor is capable of detecting one or more physiological (e.g., contact, fluid flow, pressure and/or temperature) and/or locational (e.g., location within the subject) parameters. Within further embodiments the step of detecting is a series of detections over time, and optionally, the method may further comprise the step of determining the rate of degradation of the stent, and/or estimating the time for complete degradation of the stent. Within still further embodiments, the stent can determine luminal coverage of the device by healing tissue and therefore confirm that the stent is embedded within the vessel wall (reducing or eliminating the possibility that stent fragments are released into the luminal fluids).

Within one embodiment the biodegradable stent is an esophageal, ureteral, urethral, sinus, vascular, or prostatic stent and degradation of the stent can be monitored by detecting the loss or movement of sensors over a period of time.

### E. STENT COATINGS

Within certain embodiments of the invention the stents provided herein can have one or more coatings on one or more surfaces of the stent. Coatings can be provided on stents for a variety of purposes. Coatings may be biodegradable, or non-biodegradable, or a combination of these. Typically, many coatings are polymer-based (e.g., polymers comprised of polyurethane, polyester, polylactic acid, polyamino acid, polytetrafluroethylene, tephlon, Gortex^{®}), although non-polymer coatings may also be utilized.

Representative examples of suitable coatings include those described in, for example, US Patent Nos. 8,123,799, 8,080,051, 8,001,925, 7,553,923, and 5,779,729, all of which are incorporated by reference in their entirety.

### F. DRUG -ELUTING STENTS

Within certain embodiments of the invention the stents provided herein may be designed to elute one or more drugs (e.g., biologically active agents). Representative examples include US Patent. No. 5,716,981; US Patent App. Nos. 2005/0021126 and 2005/0171594 entitled "Stents with bioactive coatings"; and US Patent App. Nos. 2005/0181005 and 2005/0181009 entitled "Implantable sensors and implantable pumps and anti-scarring agents), all of which are incorporated by reference in their entirety.

Hence, within various embodiments of the invention drug-eluting stents (e.g., a drug-coated stent) are provided which comprise one or more sensors, and which can be utilized to release a desired agent (e.g., a drug or therapeutic agent) to a desired location within the body (e.g., a body lumen and/or vessel walls). Within related embodiments, a drug-eluting delivery device may be included within the stent in order to release a desired drug upon demand (e.g., upon remote activation / demand, or based upon a timed schedule, see generally U.S. Patent App. No. 2011/0092948 entitled "Remotely Activated Piezoelectric Pump For Delivery of Biological Agents to the Intervertebral Disc and Spine", which is incorporated by reference in its entirety), or upon detection of an activating event (e.g., detection of a leak by a pressure sensor). For example, within certain embodiments of the invention biological agents can be administered along with or released from a stent in order to treat or prevent disease (e.g., i) in the case of cancer with a chemotherapeutic agent, or in the case of preventing restenosis, ii) in the case of preventing restenosis, with an anti-restenotic agent such as a taxane or a limus drug; and iii) in the case of infection, with an anti-microbial drug).

Within preferred embodiments one or more sensors (e.g., pressure sensors, contact sensors, and/or position sensors) can be utilized to determine appropriate placement of the desired drug, as well as the quantity and release kinetics of drug to be released at a desired site.

### G. METHODS FOR MONITORING INFECTION

Within other embodiments stents are provided comprising one or more temperature sensors. Such stents can be utilized to measure the temperature of blood, vessel or lumen wall, the stent, and in the local tissue and environment adjacent to the stent. Methods are also provided for monitoring changes in temperature over time, in order to determine and /or provide notice (e.g., to a patient and/or a healthcare provider) that an infection may be imminent.

In certain embodiments of the present invention, metabolic and physical sensors can also be placed on or within the stent or various components of a stent in order to monitor for rare, but potentially life-threatening complications. In some patients, the stent and surrounding tissues can become infected. Sensors such as temperature sensors (detecting temperature increases), pH sensors (detecting pH decreases), and other metabolic sensors can be used to suggest the presence of infection on or around the stent. For example, temperature sensors may be included on or within a stent in order to allow early detection of infection, and preemptive treatment with antibiotics or surgical intervention.

### H. FURTHER USES OF SENSOR-CONTAINING STENTS IN HEALTHCARE

Sensors on stents, and any associated medical device has a variety of benefits in the healthcare setting, and in non-healthcare settings (e.g., at home or work). For example,, postoperative progress can be monitored (readings compared from day-to-day, week-to-week, etc.) and the information compiled and relayed to both the patient and the attending physician allowing rehabilitation to be followed sequentially and compared to expected (typical population) norms. Within certain embodiments, a wearable device interrogates the sensors on a selected or randomized basis, and captures and /or stores the collected sensor data. This data may then be downloaded to another system or device (as described in further detail below).

Integrating the data collected by the sensors described herein (e.g., contact sensors, position sensors, strain gauges and/or accelerometers) with simple, widely available, commercial analytical technologies such as pedometers and global positioning satellite (GPS) capability, allows further clinically important data to be collected such as, but not restricted to: extent of patient ambulation (time, distance, steps, speed, cadence), patient activity levels (frequency of activity, duration, intensity), exercise tolerance (work, calories, power, training effect), range of motion and stent performance under various "real world" conditions. It is difficult to overstate the value of this information in enabling better management of the patient's recovery. An attending physician (or nurse, physiotherapist, or rehabilitation specialist) only observes the patient episodically during scheduled visits; the degree of patient function at the exact moment of examination can be impacted by a multitude of disparate factors such as: the presence or absence of pain, the presence or absence of inflammation, time of day, compliance and timing of medication use (pain medications, anti-inflammatories), recent activity, patient strength, mental status, language barriers, the nature of their doctor-patient relationship, or even the patient's ability to accurately articulate their symptoms - to name just a few. Continuous monitoring and data collection can allow the patient and the physician to monitor progress objectively by supplying objective information about patient function under numerous conditions and circumstances, to evaluate how performance has been affected by various interventions (pain control, anti-inflammatory medication, rest, etc.), and to compare patient progress versus previous function and future expected function. Better therapeutic decisions and better patient compliance can be expected when both the doctor and the patient have the benefit of observing the impact of various treatment modalities on patient rehabilitation, activity, function and overall performance.

### I. GENERATION OF POWER

Within certain aspects of the invention, one or more small electrical generation units can be positioned inside, within, and/or upon of the stent. Briefly, a variety of techniques have been described for scavenging power from small mechanical movements or mechanical vibration. See, for example, the article entitled "Piezoelectric Power Scavenging of Mechanical Vibration Energy," by U.K. Singh et al., as published in the Australian Mining Technology Conference, October 2-4, 2007, pp. 111-118, and the article entitled "Next Generation Micro-power Systems by Chandrakasan et al., as published in the 2008 Symposium on VLSI Circuits Digest of Technical Papers, pp. 1-5. See also U.S. Patent No. 8,283,793 entitled "Device for Energy Harvesting within a Vessel," and U.S. Patent No. 8,311,632 entitled "Devices, Methods and Systems for Harvesting Energy in the Body," all of the above of which are incorporated by reference in their entirety. These references provide examples of different types of power scavengers which can produce electricity from very small motion and store the electricity for later use. The above references also describes embodiments in which pressure is applied and released from the particular structure in order to produce electricity without the need for motion, but rather as a result of the application of high pressure. In addition, these references describe embodiments wherein electricity can be produced from pulsatile forces within the body.

After the electricity is generated by one or more generators, the electricity can be transmitted to any one of the variety of sensors which is described herein. For example, it can be transmitted to the sensors shown in the Figures. It may also be transmitted to the other sensors described herein. The transmission of the power can be carried out by any acceptable technique. For example, if the sensor is physically coupled to the stent, electric wires may run from the generator to the particular sensor. Alternatively, the electricity can be transmitted wirelessly in the same way that wireless smartcards receive power from closely adjacent power sources using the appropriate send and receive antennas. Such send and receive techniques of electric power are also described in the publication and the patent applications and issued U.S. patent previously described, all of which are incorporated herein by reference.

### J. MEDICAL IMAGING AND SELF-DIAGNOSIS OF ASSEMBLIES COMPRISING STENTS; PREDICTIVE ANALYSIS AND PREDICTIVE MAINTENANCE

The present invention provides stents which are capable of imaging through the use of sensors a wide variety of conditions. For example, within various aspects of the invention methods are provided for imaging a stent, or an assembly comprising a stent with sensors, comprising the steps of detecting the changes in sensors in, on, and or within a stent over time, and wherein the stent comprises sensors at a density of greater than 1, 2, 3, 4, 5, 6, 7. 8. 9. 10 or 20 sensors per square centimeter. Within other aspects the stent comprises sensors at a density of greater than 1, 2, 3, 4, 5, 6, 7, 8, 9, 10 or 20 sensors per cubic centimeter. Within either of these embodiments there can be less than 50, 75, 100, or 200 sensors per square centimeter, or per cubic centimeter. As noted above, a wide variety of sensors can be utilized therein, including for example, fluid pressure sensors, contact sensors, position sensors, pressure sensors, blood volume sensors, blood flow sensors, blood chemistry sensors, blood metabolic sensors, mechanical stress sensors, and temperature sensors

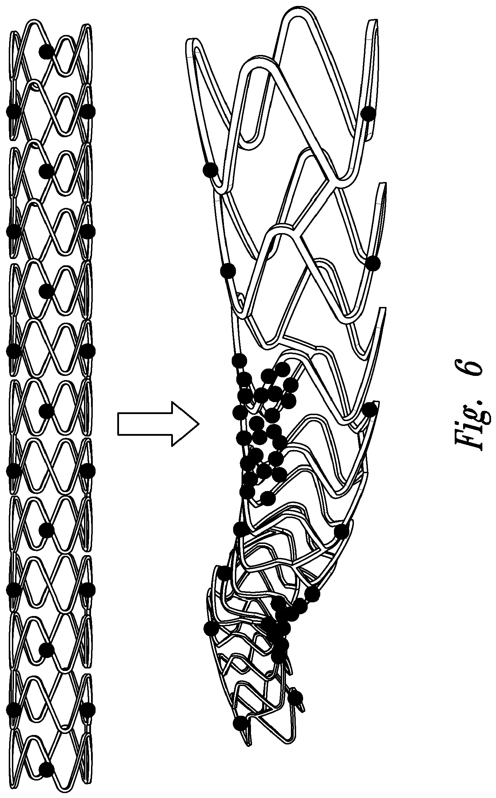

For example, as shown in Figure 6, a stent comprising sensors as described herein can be utilized to image vascular anatomy and stent anatomy through sensors which can detect positional movement. The sensors used can also include accelerometers and motion sensors to detect movement of the stent due to heart beats or other physical changes. Changes in the position of the accelerometers and/or motion sensors over time can be used as a measurement of changes in the position of the stent wall and/or vascular wall over time. Such positional changes can be used as a surrogate marker of vascular and stent anatomy - i.e. they can form an "image' of the stent and/or vascular wall to provide information on the size, shape and location of restenosis within the stent; size, shape and location of clot formation, tumor growth, abscess formation, or atherosclerotic plaque formation; kinking of the stent, stent fracture, disarticulation of a segmented or bifurcated stent, amount of overlap in overlapping stents, and/or stent movement/migration.

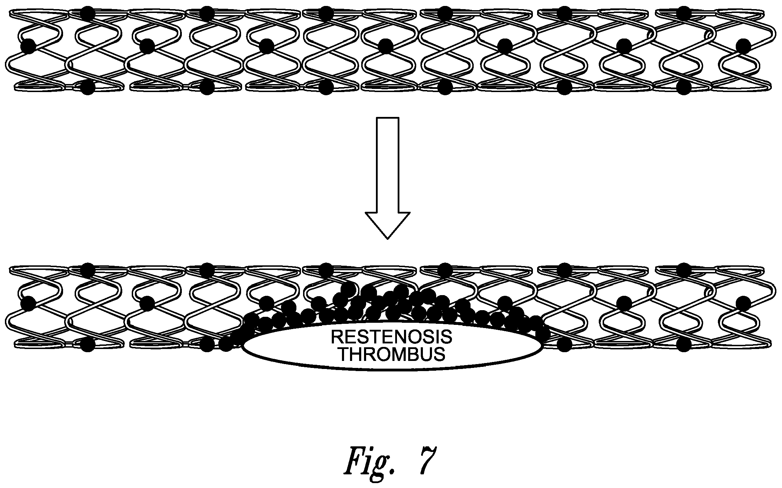

For example, Figure 7 illustrates the medical imaging of vasculature by sensors which can detect positional movement due to vascular pathology, e.g., restenosis or thrombus formation. By imaging the stent in this manner, the integrity of the stent can be wirelessly interrogated and the results reported on a regular basis. This permits the health of the subject to be checked on a regular basis or at any time as desired by the subject and/or physician, and hence, allows for predictive analysis and/or predictive maintenance or prevention of a stent.

Certain exemplary embodiments will now be explained in more detail. One particular benefit is the live and in-situ monitoring of the patient's recovery with a stent implant. The sensors as described herein can collect data on a constant basis, during normal daily activities and even during the night if desired. For example, the contact sensors can obtain and report data once every 10 seconds, once a minute, or once a day. Other sensors will collect data more frequently, such as several times a second. For example, it would be expected that the temperature, contact, and /or position data could be collected and stored several times a second. Other types of data might only need to be collected by the minute or by the hour. Still other sensors may collect data only when signaled by the patient to do so (via an external signaling/triggering device) as part of "event recording" - i.e. when the patient experiences a particular event (e.g. pain, injury, etc.) - and signals the device to obtain a reading at that time in order to allow the comparison of subjective/symptomatic data to objective/sensor data in an effort to better understand the underlying cause or triggers of the patient's symptoms.

In certain instances the stent is of sufficient size and has more than sufficient space in order to house one or more processor circuits, CPUs, memory chips and other electrical circuits as well as antennas for sending and receiving the data. Within other embodiments, the associated medical device may be able to house the one or more processor circuits, CPUs, memory chips and other electrical circuits as well as antennas for sending and receiving the data. Processors can be programmed to collect data from the various sensors on any desired schedule as set by the medical professional. All activity can be continuously monitored post operation or post-procedure and the data collected and stored in the memory located inside the stent.

A patient with a stent will generally have regular medical checkups. When the patient goes to the doctor's office for a medical checkup, the doctor will bring a reading device closely adjacent to the stent, in this example the stent, in order to transfer the data from the internal circuit inside the stent to the database in the physician's office. The use of wireless transmission using smartcards or other techniques is very well known in the art and need not be described in detail. Examples of such wireless transmission of data are provided in the published patent applications and patents which have been described herein. The data which has been collected (e.g., over a short period of time, over several weeks or even several months) is transferred in a few moments from the memory which is positioned in the stent to the doctor's computer or wireless device. The computer therefore analyzes the data for anomalies, unexpected changes over time, positive or negative trends, and other signs which may be indicative of the health of the patient and the operability of the stent. For example, if the patient has decided to go skiing or jogging, the doctor will be able to monitor the effect of such activity on the stent, including changes during such activities. The doctor can then look at the health of the stent in the hours and days after the event and compare it to data prior to the event to determine if any particular event caused long term damage, or if the activities subjected the stent to forces beyond the manufacturer's performance specifications for that particular stent. Data can be collected and compared with respect to the ongoing and long term performance of the stent from the strain gauges, the contact sensors, the surface wear sensors, or other sensors which may be present. One representative example of an electronic data capture, documentation and clinical decision support system (EDDS) is provided in WO 2012/061825, which is incorporated by reference in its entirety.

In one alternative, the patient may also have such a reading device in their home which collates the data from the stent on a periodic basis, such as once per day or once per week. As described above, the patient may also be able to "trigger" a device reading (via an external signaling/triggering device) as part of "event recording." Empowering the patient to follow their own rehabilitation - and enabling them to see the positive (and negative) effects of various lifestyle choices on their health and rehabilitation - can be expected to improve compliance and improve patient outcomes. Furthermore, their experience can be shared via the web with other patients to compare their progress versus expected "norms" for function and rehabilitation and alert them to signs and symptoms that should be brought to their doctor's attention. The performance of different stents can be compared in different patients (different sexes, weights, activity levels, etc.) to help manufacturers design better devices and assist surgeons and other healthcare providers in the selection of the right stent for specific patient types. Payers, patients, manufacturers and physicians could all benefit from the collection of this comparative information. Lastly, data accumulated at home can be collected and transmitted via the Internet to the physician's office for analysis - potentially eliminating unnecessary visits in some cases and encouraging immediate medical follow-up in others.

### K. METHODS OF MONITORING ASSEMBLIES COMPRISING A STENTS

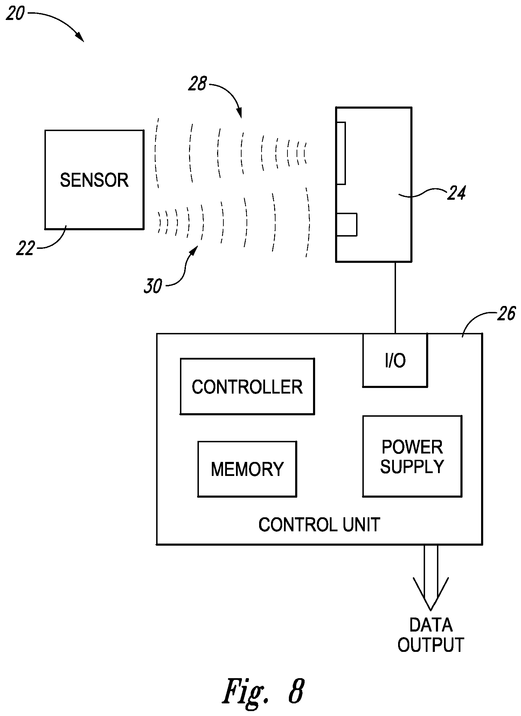

As noted above, the present invention also provides methods for monitoring one or more of the stent assemblies provided herein. For example, Figure 8 illustrates a monitoring system 20 usable with the stent 14 as of the type shown in any one of Figures 1, 2, 3, 4, 5, 6, or 7. The monitoring system 20 includes a sensor 22, an interrogation module 24, and a control unit 26. The sensor 22 can be of the passive, wireless type which can operate on power received from a wireless source. Such sensors of this type are well known in the art and widely available. A pressure sensor of this type might be a MEMS pressure sensor, for example, Part No. LPS331AP, sold on the open market by STMicroelectronics. MEMS pressure sensors are well known to operate on very low power and suitable to remain unpowered and idle for long periods of time. They can be provided power wirelessly on an RF signal and, based on the power received wirelessly on the RF signal, perform the pressure sensing and then output the sensed data.

In one embodiment, an electrical generation system is provided that can be utilized to power the sensors described herein (including for example, fluid pressure sensors, contact sensors, position sensors, pulse pressure sensors, blood volume sensors, blood flow sensors, blood chemistry sensors, blood metabolic sensors, accelerometers, mechanical stress sensors, temperature sensors, and the like). For example, the electrical generation system can rely on the pulsatile blood flow throughout a vessel. After the electricity is generated by one or more generators, it can be transmitted to any one of the variety of sensors which is described herein. The transmission of the power can be carried out by any acceptable technique. For example, the generator can be directly coupled by electrical wires to one or more sensors. Alternatively (or, in addition), the electricity can be transmitted wirelessly in the same way that wireless smartcards receive power from closely adjacent power sources using the appropriate send and receive antennas.

During operation, as shown in Figure 8, an interrogation module 24 outputs a signal 28. The signal 28 is a wireless signal (e.g., in the RF band), that contains power for the sensor 22 as well as an interrogation request that the sensors 22 perform a sensing. Upon being interrogated with the signal 28, the sensor 22 powers up and stores power in onboard capacitors sufficient to maintain operation during the sensing and data reporting. Such power receiving circuits and storing on onboard capacitors are well known in the art and therefore need not be shown in detail. The appropriate sensing is carried out by the sensor 22 and then the data is output from the sensor back to the interrogation module 24 on a signal 30, where it is received at an input port of the integration module.

According to one embodiment, sufficient signal strength is provided in the initial signal 28 to provide power for the sensor and to carry out the sensing operation and output the signal back to the interrogation module 24. In other embodiments, two or more signals 28 are sent, each signal providing additional power to the sensor to permit it to complete the sensing operation and then provide sufficient power to transfer the data via the signal path 30 back to the interrogation module 24. For example, the signal 28 can be sent continuously, with a sensing request component at the first part of the signal and then continued providing, either as a steady signal or pulses to provide power to operate the sensor. When the sensor is ready to output the data, it sends a signal alerting the interrogation module 24 that data is coming and the signal 28 can be turned off to avoid interference. Alternatively, the integration signal 28 can be at a first frequency and the output signal 30 at a second frequency separated sufficiently that they do not interfere with each other. In a preferred embodiment, they are both the same frequency so that the same antenna on the sensor can receive the signal 28 and send signal 30.

The interrogation signal 28 may contain data to select specific sensors on the stent. For example, the signal 28 may power up all sensors on the stent at the same time and then send requests for data from each at different selected times so that with one interrogation signal 28 provided for a set time, such as 1-2 seconds, results in each of the sensors on the stent collecting data during this time period and then, at the end of the period, reporting the data out on respective signals 30 at different times over the next 0.5 to 2 seconds so that with one interrogation signal 28, the data from all sensors 22 is collected.

The interrogation module 24 is operating under control of the control unit 26 which has a microprocessor for the controller, a memory, an I/O circuit to interface with the interrogation module and a power supply. The control unit may output data to a computer or other device for display and use by the physician to treat the subject.

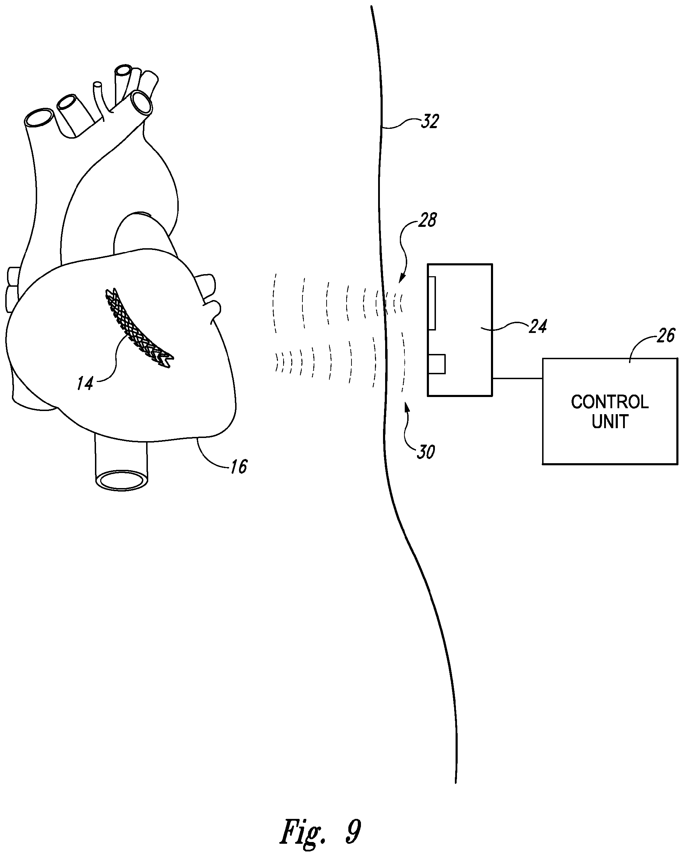

Figure 9 illustrates the operation according to a preferred embodiment within a subject. The subject has an outer skin 32. The stent placed in one of the blood vessels of the heart is located inside the body of the subject. The stent 14 may be located at any one of number of locations in the subject. In this example the stent is a coronary stent placed in the coronary artery (left anterior descending artery) of the patient; however stents in other blood vessels and nonvascular stents (as described above) could be utilized in a similar manner.

As illustrated in Figure 9, the interrogation module 24 and control unit 26 are positioned outside the skin 32 of the subject. The interrogation signal 28 passes through the skin of the subject with a wireless RF signal, and the data is received on a wireless RF signal 30 from the sensor 22 back to the interrogation module 24. While the wireless signal can be in any frequency range, an RF range is preferred. A frequency in the VLF to LF ranges of between 3-300 kHz is preferred to permit the signal to be carried to sufficient depth inside the body with low power, but frequencies below 3 kHz and above 300 kHz can also be used. The sensing does not require a transfer of large amounts of data and low power is preferred; therefore, a low frequency RF signal is acceptable. This also avoids competition from and inadvertent activation by other wireless signal generators, such as blue tooth, cell phones and the like.

### L. COLLECTION, TRANSMISSION, ANALYSIS, AND DISTRIBUTION OF DATA FROM ASSEMBLIES COMPRISING STENTS

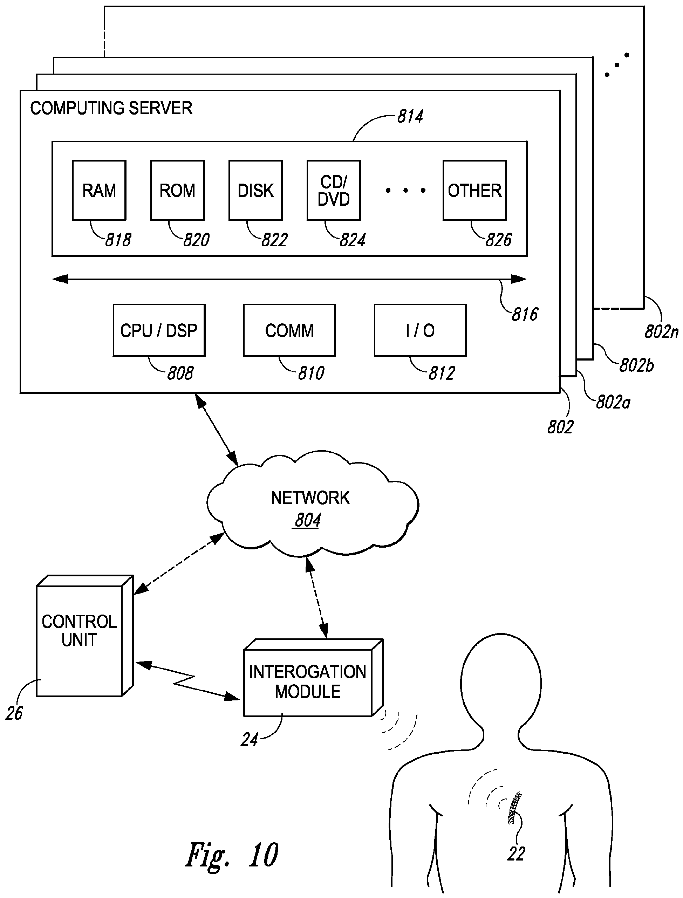

Figure 10 illustrates one embodiment of an information and communication technology (ICT) system 800 arranged to process sensor data (*e.g.,* data from sensor 22 of any one of Figures 1, 2, 3 4, 5, 6, or 7). In Figure 10, the ICT system 800 is illustrated to include computing devices that communicate via a network 804, however in other embodiments, the computing devices can communicate directly with each other or through other intervening devices, and in some cases, the computing devices do not communicate at all. The computing devices of Figure 10 include computing servers 802, control units 26, interrogation units 24, and other devices that are not shown for simplicity.

In Figure 10, one or more sensors 22 communicate with an interrogation module 24. The interrogation module 24 of Figure 10 is directed by a control unit 26, but in other cases, interrogation modules 24 operates autonomously and passes information to and from sensors 22. One or both of the interrogation module 24 and control unit 26 can communicate with the computing server 802.

Within certain embodiments, the interrogation module and/or the control unit may be a wearable device on the subject. The wearable device (e.g., a watch-like device, a wrist-band, glasses, or other device that may be carried or worn by the subject) can interrogate the sensors over a set (or random) period of time, collect the data, and forward the data on to one or more networks (804). Furthermore, the wearable device may collect data of its own accord which can also be transmitted to the network. Representative examples of data that may be collected include location (e.g., a GPS), body or skin temperature, and other physiologic data (e.g., pulse). Within yet other embodiments, the wearable device may notify the subject directly of any of a number of prescribed conditions, including but not limited to possible or actual failure of the device.

The information that is communicated between an interrogation module 24 and a sensor 22 may be useful for many purposes as described herein. In some cases, for example, sensor data information is collected and analyzed expressly for the health of an individual subject. In other cases, sensor data is collected and transmitted to another computing device to be aggregated with other data (for example, the sensor data from 22 may be collected and aggregated with other data collected from a wearable device (e.g., a device that may, in certain embodiments, include GPS data and the like).

Figure 10 illustrates aspects of a computing server 802 as a cooperative bank of servers further including computing servers 802a, 802b, and one or more other servers 802n. It is understood that computing server 802 may include any number of computing servers that operate individually or collectively to the benefit of users of the computing servers.

In some embodiments, the computing servers 802 are arranged as cloud computing devices created in one or more geographic locations, such as the United States and Canada. The cloud computing devices may be created as MICROSOFT AZURE cloud computing devices or as some other virtually accessible remote computing service.

An interrogation module 24 and a control unit 26 are optionally illustrated as communicating with a computing server 802. Via the interrogation module 24 or control unit 26, sensor data is transferred to (and in addition or alternatively from) a computing server 802 through network 804.

The network 804 includes some or all of cellular communication networks, conventional cable networks, satellite networks, fiber-optic networks, and the like configured as one or more local area networks, wide area networks, personal area networks, and any other type of computing network. In a preferred embodiment, the network 804 includes any communication hardware and software that cooperatively works to permit users of computing devices to view and interact with other computing devices.

Computing server 802 includes a central processing unit (CPU) digital signal processing unit (DSP) 808, communication modules 810, Input/Output (I/O) modules 812, and storage module 814. The components of computing server 802 are cooperatively coupled by one or more buses 816 that facilitate transmission and control of information in and through computing server 802. Communication modules 810 are configurable to pass information between the computer server 802 and other computing devices (e.g., computing servers 802a, 802b, 802n, control unit 26, interrogation unit 24, and the like). I/O modules 812 are configurable to accept input from devices such as keyboards, computer mice, trackballs, and the like. I/O modules 812 are configurable to provide output to devices such as displays, recorders, LEDs, audio devices, and the like.

Storage module 814 may include one or more types of storage media. For example, storage module 814 of Figure 10 includes random access memory (RAM) 818, read only memory (ROM) 820, disk based memory 822, optical based memory 824, and other types of memory storage media 826. In some embodiments one or more memory devices of the storage module 814 has configured thereon one or more database structures. The database structures may be used to store data collected from sensors 22.

In some embodiments, the storage module 814 may further include one or more portions of memory organized a non-transitory computer-readable media (CRM). The CRM is configured to store computing instructions executable by a CPU 808. The computing instructions may be stored as one or more files, and each file may include one or more computer programs. A computer program can be standalone program or part of a larger computer program. Alternatively or in addition, each file may include data or other computational support material for an application that directs the collection, analysis, processing, and/or distribution of data from sensors (e.g., stent sensors). The sensor data application typically executes a set of instructions stored on computer-readable media.

It will be appreciated that the computing servers shown in the figures and described herein are merely illustrative and are not intended to limit the scope of the present invention. Computing server 802 may be connected to other devices that are not illustrated, including through one or more networks such as the Internet or via the Web that are incorporated into network 804. More generally, a computing system or device (e.g., a "client" or "server") or any part thereof may comprise any combination of hardware that can interact and perform the described types of functionality, optionally when programmed or otherwise configured with software, including without limitation desktop or other computers, database servers, network storage devices and other network devices, PDAs, cell phones, wireless phones, pagers, electronic organizers, Internet appliances, television-based systems (e.g., using set-top boxes and/or personal/digital video recorders), and various other products that include appropriate inter-communication capabilities. In addition, the functionality provided by the illustrated system modules may in some embodiments be combined in fewer modules or distributed in additional modules. Similarly, in some embodiments the functionality of some of the illustrated modules may not be provided and/or other additional functionality may be available.

In addition, while various items are illustrated as being stored in memory or on storage while being used, these items or portions of them can be transferred between memory and other storage devices for purposes of memory management and/or data integrity. In at least some embodiments, the illustrated modules and/or systems are software modules/systems that include software instructions which, when executed by the CPU/DSP 808 or other processor, will program the processor to automatically perform the described operations for a module/system. Alternatively, in other embodiments, some or all of the software modules and/or systems may execute in memory on another device and communicate with the illustrated computing system/device via inter-computer communication.

Furthermore, in some embodiments, some or all of the modules and/or systems may be implemented or provided in other manners, such as at least partially in firmware and/or hardware means, including, but not limited to, one or more application-specific integrated circuits (ASICs), standard integrated circuits, controllers (e.g., by executing appropriate instructions, and including microcontrollers and/or embedded controllers), field-programmable gate arrays (FPGAs), complex programmable logic devices (CPLDs), and the like. Some or all of the systems, modules, or data structures may also be stored *(e.g.,* as software instructions or structured data) on a transitory or non-transitory computer-readable storage medium 814, such as a hard disk 822 or flash drive or other non-volatile storage device 826, volatile 818 or non-volatile memory 820, a network storage device, or a portable media article (*e.g.,* a DVD disk, a CD disk, an optical disk, a flash memory device, etc.) to be read by an appropriate input or output system or via an appropriate connection. The systems, modules, and data structures may also in some embodiments be transmitted as generated data signals (e.g., as part of a carrier wave or other analog or digital propagated signal) on a variety of computer readable transmission mediums, including wireless-based and wired/cable-based mediums. The data signals can take a variety of forms such as part of a single or multiplexed analog signal, as multiple discrete digital packets or frames, as a discrete or streaming set of digital bits, or in some other form. Such computer program products may also take other forms in other embodiments. Accordingly, the present invention may be practiced with other computer system configurations.

In Figure 10, sensor data from, e.g., sensor 22 is provided to computing server 802. Generally speaking, the sensor data, represents data retrieved from a known subject and from a known sensor. The sensor data may possess include or be further associated with additional information such as the USI, UDI, a time stamp, a location (e.g., GPS) stamp, a date stamp, and other information. The differences between various sensors is that some may include more or fewer data bits that associate the data with a particular source, collection device, transmission characteristic, or the like.

In some embodiments, the sensor data may comprise sensitive information such as private health information associated with a specific subject. Sensitive information, for example sensor data from sensor 22, may include any information that an associated party desires to keep from wide or easy dissemination. Sensitive information can stand alone or be combined with other non-sensitive information. For example, a subject's medical information is typically sensitive information. In some cases, the storage and transmission of a subject's medical information is protected by a government directive (e.g., law, regulation, etc.) such as the U.S. Health Insurance Portability and Accountability Act (HIPPA).

As discussed herein, a reference to "sensitive" information includes information that is entirely sensitive and information that is some combination of sensitive and non-sensitive information. The sensitive information may be represented in a data file or in some other format. As used herein, a data file that includes a subject's medical information may be referred to as "sensitive information." Other information, such as employment information, financial information, identity information, and many other types of information may also be considered sensitive information.

A computing system can represent sensitive information with an encoding algorithm (e.g., ASCII), a well-recognized file format (e.g., PDF), or by some other format. In a computing system, sensitive information can be protected from wide or easy dissemination with an encryption algorithm.

Generally speaking, sensitive information can be stored by a computing system as a discrete set of data bits. The set of data bits may be called "plaintext." Furthermore, a computing system can use an encryption process to transform plaintext using an encryption algorithm (i.e., a cipher) into a set of data bits having a highly unreadable state (i.e., cipher text). A computing system having knowledge of the encryption key used to create the cipher text can restore the information to a plaintext readable state. Accordingly, in some cases, sensitive data (e.g., sensor data 806a, 806b) is optionally encrypted before being communicated to a computing device.

In one embodiment, the operation of the information and communication technology (ICT) system 800 of Figure 10 includes one or more sensor data computer programs stored on a computer-readable medium. The computer program may optionally direct and/or receive data from one or more stent sensors implanted in one or more subjects. A sensor data computer program may be executed in a computing server 802. Alternatively, or in addition, a sensor data computer program may be executed in a control unit 26, an interrogation unit 24.

In one embodiment, a computer program to direct the collection and use of stent sensor data is stored on a non-transitory computer-readable medium in storage module 814. The computer program is configured to identify a subject who has a wireless stent inserted in his or her body. The wireless stent may include one or more wireless sensor

In some cases, the computer program identifies one subject, and in other cases, two or more subjects are identified. The subjects may each have one or more wireless stents, and each wireless stent may have one or more wireless sensors of the type described herein.

The computer program is arranged to direct the collection of sensor data from the wireless stent devices. The sensor data is generally collected with a wireless interrogation unit 24. In some cases, the program communicates with the wireless interrogation unit 24. In other cases, the program communicates with a control unit 26, which in turn directs a wireless interrogation unit 24. In still other cases, some other mechanism is used direct the collection of the sensor data.

Once the sensor data is collected, the data may be further processed. For example, in some cases, the sensor data includes sensitive subject data, which can be removed or disassociated with the data. The sensor data can be individually stored (e.g., by unique sensor identification number, device number, etc.) or aggregated together with other sensor data by sensor type, time stamp, location stamp, date stamp, subject type, other subject characteristics, or by some other means.

The following pseudo-code description is used to generally illustrate one exemplary algorithm executed by a computing server 802 and generally described herein with respect to Figure 10:

```
         Start
         Open a secure socket layer (SSL)
         Identify a subject
         Communicate with a predetermined control unit
         Request sensor data from the subject via the control unit
         Receive sensor data
         If the sensor data is encrypted
                THEN decrypt the sensor data
         Store encrypted data in the selected storage locations
         Aggregate the sensor data with other sensor data
         Store encrypted data in the selected storage locations
         Maintain a record of the storage transaction
         Perform post storage actions
 End
```

Those skilled in the art will recognize that it is common within the art to implement devices and/or processes and/or systems, and thereafter use engineering and/or other practices to integrate such implemented devices and/or processes and/or systems into more comprehensive devices and/or processes and/or systems. That is, at least a portion of the devices and/or processes and/or systems described herein can be integrated into other devices and/or processes and/or systems via a reasonable amount of experimentation. Those having skill in the art will recognize that examples of such other devices and/or processes and/or systems might include-as appropriate to context and application-all or part of devices and/or processes and/or systems of (a) an air conveyance (e.g., an airplane, rocket, helicopter), (b) a ground conveyance (e.g., a car, truck, locomotive, tank, armored personnel carrier), (c) a building (e.g., a home, warehouse, office, ), (d) an appliance (e.g., a coffee machine, refrigerator, a washing machine, a dryer), (e) a communications system (e.g., a networked system, a telephone system, a Voice over IP system), (f) a business entity (e.g., an Internet Service Provider (ISP) entity such as Comcast Cable, Qwest, Southwestern Bell), or (g) a wired/wireless services entity (e.g., AT&T, T-Mobile, Verizon).

In certain cases, use of a system or method may occur in a territory even if components are located outside the territory. For example, in a distributed computing context, use of a distributed computing system may occur in a territory even though parts of the system may be located outside of the territory (e.g., relay, server, processor, signal-bearing medium, transmitting computer, receiving computer, etc. located outside the territory). Within one embodiment of the invention, a subject having a stent may be in one location, while processing and analysis of the data is performed in another location.

A sale of a system or method may likewise occur in a territory even if components of the system or method are located and/or used outside the territory. Further, implementation of at least part of a system for performing a method in one territory does not preclude use of the system in another territory.

In conclusion, stents utilizing a variety of sensors can be utilized to serve a variety of critical clinical functions, such as safe, accurate and less traumatic placement and deployment of the stent, procedural and post-operative "real time" imaging of stent and the surrounding anatomy, the development of stent complications, and the patient's overall health status (cardiac, renal and other physiologic parameters). Currently, post-operative (both in hospital and out-patient) evaluation of stent patients is through patient history, physical examination and medical monitoring (vital signs, blood work, ECG, etc.) that is supplemented with diagnostic imaging studies as required. However, most of the patient's recuperative period occurs between hospital and office visits and the majority of data on daily function goes uncaptured; furthermore, monitoring patient progress through the use of some diagnostic imaging technology can be expensive, invasive and carry its own health risks (coronary angiography for example). It can, therefore, be very difficult to accurately measure and follow the development or worsening of symptoms and evaluate "real life" stent performance, particularly as they relate to patient activity levels, exercise tolerance, and the effectiveness of rehabilitation efforts and medications.

At present, neither the physician nor the patient has access to the type of "real time," continuous, objective, stent performance measurements that they might otherwise like to have. Being able to monitor *in situ* stent function, integrity, anatomy and physiology can provide the physician with valuable objective information during office visits; furthermore, the patient can take additional readings at home at various times (e.g. when experiencing pain, during exercise, after taking medications, etc.) to provide important complementary clinical information to the doctor (which can be sent to the healthcare provider electronically even from remote locations). From the perspective of the patient, being able to monitor many of these same parameters at home allows them to take a more proactive role in their care and recovery and provide him or her with either an early warning indicator to seek medical assistance or with reassurance.

In one alternative, the patient may have a reading device in their home which collates the data from the stent on a periodic basis, such as once per day or once per week. In addition to empowering the patient to follow their own rehabilitation - and enabling them to see the positive (and negative) effects of various lifestyle choices on their health and rehabilitation - such information access can be expected to improve compliance and improve patient outcomes. For example, within certain embodiments the devices and systems provided herein can instruct or notify the patient, or a permitted third-party as to deviations (e.g., greater than 10%, 20%, 25%, 50%, 70%, and or 100%) from normal, and/or, set parameters. Furthermore, their recovery experience can be shared via the web with other patients to compare their progress versus expected "norms" for function and rehabilitation and alert them to signs and symptoms that should be brought to their doctor's attention (e.g., on Facebook or other social media sites). From a public health perspective, the performance of different stents can be compared in different patients (different sexes, disease severity, activity levels, concurrent diseases such as hypertension and diabetes, smoking status, obesity, etc.) to help manufacturers design better stents and assist physicians in the selection of the right stent for a specific patient types. Payers, patients, manufacturers and physicians could all benefit from the collection of this comparative information. Poor and dangerous products could be identified and removed from the market and objective long-term effectiveness data collected and analyzed. Lastly, data accumulated at home can be collected and transmitted via the Internet to the physician's office for analysis - potentially eliminating unnecessary visits in some cases and encouraging immediate medical follow-up in others.

The following are some specific numbered embodiments of the systems and processes disclosed herein. These embodiments are exemplary only. It will be understood that the invention is not limited to the embodiments set forth herein for illustration, but embraces all such forms thereof as come within the scope of the above disclosure.
1) An assembly comprising a stent; and a sensor positioned on or within said stent.
2) The assembly according to embodiment 1 wherein the sensor is positioned on an outer wall of the stent.
3) The assembly according to embodiment 1 wherein the sensor is positioned on an inner wall of the stent.
4) The assembly according to embodiment 1 wherein the sensor is positioned within the stent.
5) The assembly according to embodiment 1 wherein the sensor is positioned on the luminal surface, adluminal surface, and/or implanted within a lumen.
6)The assembly according to any one of embodiments 1 to 4 wherein the sensor is a fluid pressure sensor.
7) The assembly according to any one of embodiments 1 to 4 wherein the sensor is a contact sensor.
8) The assembly according to any one of embodiments 1 to 4 wherein the sensor is a position sensor.
9) The assembly according to any one of embodiments 1 to 4 wherein the sensor is a pulse pressure sensor.
10) The assembly according to any one of embodiments 1 to 4 wherein the sensor is a blood volume sensor
11) The assembly according to any one of embodiments 1 to 4 wherein the sensor is a blood flow sensor.
12) The assembly according to any one of embodiments 1 to 4 wherein the sensor is a blood chemistry sensor.
13) The assembly according to any one of embodiments 1 to 4 wherein the sensor is a blood metabolic sensor.
14) The assembly according to any one of embodiments 1 to 4 wherein the sensor is a mechanical stress sensor, accelerometer or a temperature sensor.
15) The assembly according to any one of embodiments 1 to 14 wherein said stent is a vascular, gastrointestinal, pulmonary, head and neck, or genitourinary stent.
16) The assembly according to embodiment 15 wherein said vascular stent is a coronary stent, carotid stent, cerebral stent, vertebral stent, iliac stent, femoral stent, popliteal stent, or stent for the arteries of the lower extremities.
17) The assembly according to embodiment 15 wherein said gastrointestinal stent is an esophageal, duodenal, colonic, biliary or pancreatic stent.
18) The assembly according to embodiment 15 wherein said pulmonary stent is a stent that holds open the trachea, bronchi, bronchioles or alveoli.
19) The assembly according to embodiment 15 wherein said genitourinary stent is a ureteral stent, urethral stent, a prostatic stent, or a fallopian tube stent.
20) The assembly according to embodiment 15 wherein said head and neck stent is a sinus stent, a maxillary sinus stent, a frontal sinus stent, a lacrimal stent, a nasal stent, or a typanostomy tube.
21) The assembly according to any one of embodiments 1 to 20 wherein said stent is a biodegradable or partially biodegradable stent.
22) The assembly according to any one of embodiments 1 to 20 wherein said stent is a non-23) biodegradable stent.
23) The assembly according to any one of embodiments 1 to 22 wherein said sensor is a wireless sensor.
24) The assembly according to any one of embodiments 1 to 22 wherein said sensor is connected to a wireless microprocessor.
25) The assembly according to any one of embodiments 1 to 24 wherein a plurality of sensors are positioned on or within said stent.
26) The assembly according to any one of embodiments 1 to 25 wherein said stent comprises more than one type of sensor.
27) The assembly according to any one of embodiments 1 to 26 wherein said stent comprises one or more fluid pressure sensors, contact sensors, accelerometers, and position sensors.
28) The assembly according to any one of embodiments 1 to 27 wherein said sensor is a plurality of sensors which are positioned on or within said stent at a density of greater than 1, 2, 3, 4, 5, 6, 7, 8, 9, 10 or 20 sensors per square centimeter.
29) The assembly according to any one of embodiments 1 to 27 wherein said sensor is a plurality of sensors which are positioned on or within said stent at a density of greater than 1, 2, 3, 4, 5, 6, 7, 8, 9, 10 or 20 sensors per cubic centimeter.
30) The assembly according to any one of embodiments 1 to 29 wherein said sensor has a unique sensor identification number.
31) The assembly according to any one of embodiments 1 to 30 wherein said sensor is uniquely defined within a specific position on or within said stent.
32) The assembly according to any one of embodiments 1 to 31 wherein said stent is comprised of two or more sections.
32) The assembly according to embodiment 32 wherein sensors are positioned on each of said two or more sections.
34) The assembly according to embodiment 32 wherein said sensors can be utilized to detect proper connection or assembly of a complete stent.
35) An assembly comprising a stent and a sensor, wherein said sensor measures the cardiac output of a subject.
36) An assembly comprising a stent and a sensor, wherein said sensor measures the stroke volume of a subject.
37) An assembly comprising a stent and a sensor, wherein said sensor measures the ejection fraction of a subject.
38) An assembly comprising a stent and a sensor, wherein said sensor measures the systolic blood pressure of a subject.
39) An assembly comprising a stent and a sensor, wherein said sensor measures the diastolic blood pressure of a subject.
40) An assembly comprising a stent and a sensor, wherein said sensor measures the mean arterial pressure of a subject.
41) An assembly comprising a stent and a sensor, wherein said sensor measures the systemic vascular resistance of a subject.
42) An assembly comprising a stent and a sensor, wherein said sensor measures the total peripheral resistance of a subject.
43) An assembly comprising a stent and a sensor, wherein said sensor measures the temperature of a subject.
44) An assembly comprising a stent and a sensor, wherein said sensor measures the development of restenosis.
45) An assembly comprising a stent and a sensor, wherein said sensor measures a cardiac function.
46) An assembly comprising a stent and a sensor, wherein said sensor measures the development of a thrombus, atherosclerosis, tumor, inflammation, abscess or other space occupying lesion.
47) An assembly comprising a stent and a sensor, wherein said sensor measures the development of normal healing tissue on the luminal surface of the stent.
48) An assembly comprising a stent and a sensor, wherein said sensor measures the metabolic function including indicators of renal function.
49) An assembly comprising a stent and a sensor, wherein said sensor measures heart rhythm including conduction and rhythm abnormalities.
50) An assembly according to any one of embodiments 1 to 49 wherein said stent is a drug-eluting stent.
51) An assembly according to any one of embodiments 1 to 50 wherein said stent is at least partially coated with one or more polymers.
52) Use of a stent or assembly according to any one of embodiments 1 to 51 to obtain a measurement of cardiac function.
53) Use according to embodiment 52 wherein said measurement of cardiac function is selected from the group consisting of cardiac output, stroke volume, ejection fraction, systolic and/or diastolic blood pressure, mean arterial pressure, systemic vascular resistance, and total peripheral resistance.
54) Use according to embodiment 52 or 53, wherein said measurement occurs at more than one time point.
55) Use according to any one of embodiments 52 to 54 wherein said measurement takes place over more than 1, 2, 3, 4, 5, 10, 15, or 30 days.
56) Use according to any one of embodiments 52 to 55 wherein said measurement takes place over more than 1, 2, 3, 4, 5, 6, or 12 months.
57) A method of monitoring a stent comprising:
   transmitting a wireless electrical signal from a location outside the body to a location inside the body;
   receiving the signal at a sensor positioned on a stent located inside the body;
   powering the sensor using the received signal;
   sensing data at the sensor; and
   outputting the sensed data from the sensor to a receiving unit located outside of the body.
58) The method according to embodiment 57 wherein said stent is an assembly according to any one of embodiments 1 to 51.
59) The method according to embodiment 57 or 58 wherein said receiving unit is a watch, writs band, cell phone or glasses.
60) The method according to any one of embodiments 57 to 59 wherein said receiving unit is located within a subject's residence or office.
61) The method according to any one of embodiments 57 to 60 wherein said sensed data is provided to a health care provider.
62) The method according to any one of embodiments 57 to 61 wherein said sensed data is posted to one or more websites.
63) A non-transitory computer-readable storage medium whose stored contents configure a computing system to perform a method, the method comprising:
   identifying a subject, the identified subject having at least one wireless stent, each wireless stent having one or more wireless sensors;
   directing a wireless interrogation unit to collect sensor data from at least one of the respective one or more wireless sensors; and
   receiving the collected sensor data.
64) The non-transitory computer-readable storage medium of embodiment 63 whose stored contents configure a computing system to perform a method, the method further comprising:
   identifying a plurality of subjects, each identified subject having at least one wireless stent, each wireless stent having one or more wireless sensors;
   directing a wireless interrogation unit associated with each identified subject to collect sensor data from at least one of the respective one or more wireless sensors;
   receiving the collected sensor data; and
   aggregating the collected sensor data.
65) The non-transitory computer-readable storage medium of embodiment 63 whose stored contents configure a computing system to perform a method, the method further comprising:
   removing sensitive subject data from the collected sensor data; and
   parsing the aggregated data according to a type of sensor.
66) The non-transitory computer-readable storage medium of embodiment 63 whose stored contents configure a computing system to perform a method, wherein directing the wireless interrogation unit includes directing a control unit associated with the wireless interrogation unit.
67) The non-transitory computer readable storage medium according to any one of embodiments 63 to 66, wherein said stent is an assembly according to any one of embodiments 1 to 51.
68) The storage medium according to any one of embodiments 63 to 67 wherein said collected sensor data is received on a watch, wrist band, cell phone or glasses.
69) The storage medium according to any one of embodiments 63 to 68 wherein said collected sensor data is received within a subject's residence or office.
70) The storage medium according to any one of embodiments 63 to 69 wherein said collected sensed data is provided to a health care provider.
71) The storage medium according to any one of embodiments 63 to 70 wherein said sensed data is posted to one or more websites.
72) The method according to any one of embodiments 57 to 62, or storage medium according to any one of embodiments 63 to 71, wherein said data is analyzed.
73) The method or storage medium according to embodiment 72 wherein said data is plotted to enable visualization of change over time.
74) The method or storage medium according to embodiments 72 or 73 wherein said data is plotted to provide a three-dimensional image.
75) A method for determining degradation of a stent, comprising the steps of a) providing to a body passageway of a subject an assembly comprising a stent and one or more sensors, and b) detecting a change in a sensor, and thus determining degradation of the stent.
76) The method according to embodiment 75 wherein said sensor is capable of detecting one or more physiological and/or locational parameters.
77) The method according to embodiment 75 or 76 wherein said sensor detects contact, fluid flow, pressure and/or temperature.
78) The method according to any one of embodiments 75 to 77 wherein said sensor detects a location within the subject.
70) The method according to any one of embodiments 75 to 78 wherein said assembly is an assembly according to embodiments 1 to 51.
80) The method according to any one of embodiments 75 to 79 wherein the step of detecting is a series of detections over time.
81) A method for imaging a stent, comprising detecting the changes in sensors in, on, and or within a stent over time, and wherein the stent comprises sensors at a density of greater than 1, 2, 3, 4, 5, 6, 7, 8, 9, 10 or 20 sensors per square centimeter.
82) A method for imaging a stent, comprising detecting changes in sensors in, on, and or within a stent over time, and wherein the stent comprises sensors at a density of greater than 1, 2, 3, 4, 5, 6, 7, 8, 9, 10 or 20 sensors per cubic centimeter.
83) The method according to embodiments 81 or 82, wherein said sensor is one or more of a fluid pressure sensor, contact sensor, position sensor, accelerometer, pressure sensor, blood volume sensor, blood flow sensor, blood chemistry sensor, blood metabolic sensor, mechanical stress sensor, and temperature sensor.
84) The method according to any one of embodiments 81 to 83 wherein said stent is an assembly according to any one of embodiments 1 to 51.
85) A method for placing a stent within a subject, comprising a) implanting an assembly according to any one of embodiments 1 to 51, and b) detecting placement of the stent by detecting a sensor.
86) The method according to embodiment 85 wherein the stent comprises two or more sections, and wherein detection of said two or more sections can be determined by analysis of one or more sensors.
87) The method according to embodiments 85 or 86 wherein placement of the stent can be visualized by a two or three dimensional representation or image of the one or more sensors on said stent.
88) The method according to any one of embodiments 85 to 87, wherein said method comprises two stents which are implanted to overlap with each other.
89) The method according to anyone of embodiments 85 to 88 wherein said detecting placement of the stent allows determination of whether the stent is kinked or placed incorrectly.

Any of the various embodiments described above can be combined to provide further embodiments. All of the U.S. patents, U.S. patent application publications, U.S. patent applications, PCT application publications, foreign patents, foreign patent applications and non-patent publications referred to in this specification, are incorporated herein by reference, in their entirety. Aspects of the embodiments can be modified, if necessary to employ concepts of the various patents, applications and publications to provide yet further embodiments. These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

The invention will now be defined by reference to the following clauses:
1. An assembly comprising a stent; and a sensor positioned on or within said stent.
2. The assembly according to clause 1 wherein the sensor is positioned on an outer wall of the stent.
3. The assembly according to clause 1 wherein the sensor is positioned on an inner wall of the stent.
4. The assembly according to clause 1 wherein the sensor is positioned within the stent.
5. The assembly according to clause 1 wherein the sensor is positioned on the luminal surface, adluminal surface, and/or implanted within a lumen.
6. The assembly according to any one of clauses 1 to 4 wherein the sensor is a fluid pressure sensor.
7. The assembly according to any one of clauses 1 to 4 wherein the sensor is a contact sensor.
8. The assembly according to any one of clauses 1 to 4 wherein the sensor is a position sensor.
9. The assembly according to any one of clauses 1 to 4 wherein the sensor is a pulse pressure sensor.
10. The assembly according to any one of clauses 1 to 4 wherein the sensor is a blood volume sensor
11. The assembly according to any one of clauses 1 to 4 wherein the sensor is a blood flow sensor.
12. The assembly according to any one of clauses 1 to 4 wherein the sensor is a blood chemistry sensor.
13. The assembly according to any one of clauses 1 to 4 wherein the sensor is a blood metabolic sensor.
14. The assembly according to any one of clauses 1 to 4 wherein the sensor is a mechanical stress sensor, accelerometer or a temperature sensor.
15. The assembly according to any one of clauses 1 to 14 wherein said stent is a vascular, gastrointestinal, pulmonary, head and neck, or genitourinary stent.
16. The assembly according to clause 15 wherein said vascular stent is a coronary stent, carotid stent, cerebral stent, vertebral stent, iliac stent, femoral stent, popliteal stent, or stent for the arteries of the lower extremities.
17. The assembly according to clause 15 wherein said gastrointestinal stent is an esophageal, duodenal, colonic, biliary or pancreatic stent.
18. The assembly according to clause 15 wherein said pulmonary stent is a stent that holds open the trachea, bronchi, bronchioles or alveoli.
19. The assembly according to clause 15 wherein said genitourinary stent is a ureteral stent, urethral stent, a prostatic stent, or a fallopian tube stent.
20. The assembly according to clause 15 wherein said head and neck stent is a sinus stent, a maxillary sinus stent, a frontal sinus stent, a lacrimal stent, a nasal stent, or a typanostomy tube.
21. The assembly according to any one of clauses 1 to 20 wherein said stent is a biodegradable or partially biodegradable stent.
22. The assembly according to any one of clauses 1 to 20 wherein said stent is a non-biodegradable stent.
23. The assembly according to any one of clauses 1 to 22 wherein said sensor is a wireless sensor.
24. The assembly according to any one of clauses 1 to 22 wherein said sensor is connected to a wireless microprocessor.
25. The assembly according to any one of clauses 1 to 24 wherein a plurality of sensors are positioned on or within said stent.
26. The assembly according to any one of clauses 1 to 25 wherein said stent comprises more than one type of sensor.
27. The assembly according to any one of clauses 1 to 26 wherein said stent comprises one or more fluid pressure sensors, contact sensors, accelerometers, and position sensors.
28. The assembly according to any one of clauses 1 to 27 wherein said sensor is a plurality of sensors which are positioned on or within said stent at a density of greater than 1, 2, 3, 4, 5, 6, 7, 8, 9, 10 or 20 sensors per square centimeter.
29. The assembly according to any one of clauses 1 to 27 wherein said sensor is a plurality of sensors which are positioned on or within said stent at a density of greater than 1, 2, 3, 4, 5, 6, 7, 8, 9, 10 or 20 sensors per cubic centimeter.
30. The assembly according to any one of clauses 1 to 29 wherein said sensor has a unique sensor identification number.
31. The assembly according to any one of clauses 1 to 30 wherein said sensor is uniquely defined within a specific position on or within said stent.
32. The assembly according to any one of clauses 1 to 31 wherein said stent is comprised of two or more sections.
33. The assembly according to clause 32 wherein sensors are positioned on each of said two or more sections.
34. The assembly according to clause 32 wherein said sensors can be utilized to detect proper connection or assembly of a complete stent.
35. An assembly comprising a stent and a sensor, wherein said sensor measures the cardiac output of a subject.
36. An assembly comprising a stent and a sensor, wherein said sensor measures the stroke volume of a subject.
37. An assembly comprising a stent and a sensor, wherein said sensor measures the ejection fraction of a subject.
38. An assembly comprising a stent and a sensor, wherein said sensor measures the systolic blood pressure of a subject.
39. An assembly comprising a stent and a sensor, wherein said sensor measures the diastolic blood pressure of a subject.
40. An assembly comprising a stent and a sensor, wherein said sensor measures the mean arterial pressure of a subject.
41. An assembly comprising a stent and a sensor, wherein said sensor measures the systemic vascular resistance of a subject.
42. An assembly comprising a stent and a sensor, wherein said sensor measures the total peripheral resistance of a subject.
43. An assembly comprising a stent and a sensor, wherein said sensor measures the temperature of a subject.
44. An assembly comprising a stent and a sensor, wherein said sensor measures the development of restenosis.
45. An assembly comprising a stent and a sensor, wherein said sensor measures a cardiac function.
46. An assembly comprising a stent and a sensor, wherein said sensor measures the development of a thrombus, atherosclerosis, tumor, inflammation , abscess or other space occupying lesion.
47. An assembly comprising a stent and a sensor, wherein said sensor measures the development of normal healing tissue on the luminal surface of the stent.
48. An assembly comprising a stent and a sensor, wherein said sensor measures the metabolic function including indicators of renal function.
49. An assembly comprising a stent and a sensor, wherein said sensor measures heart rhythm including conduction and rhythm abnormalities.
50. An assembly according to any one of clauses 1 to 49 wherein said stent is a drug-eluting stent.
51. An assembly according to any one of clauses 1 to 50 wherein said stent is at least partially coated with one or more polymers.
52. Use of a stent or assembly according to any one of clauses 1 to 51 to obtain a measurement of cardiac function.
53. Use according to clause 52 wherein said measurement of cardiac function is selected from the group consisting of cardiac output, stroke volume, ejection fraction, systolic and/or diastolic blood pressure, mean arterial pressure, systemic vascular resistance, and total peripheral resistance.
54. Use according to clause 52 or 53, wherein said measurement occurs at more than one time point.
55. Use according to any one of clauses 52 to 54 wherein said measurement takes place over more than 1, 2, 3, 4, 5, 10, 15, or 30 days.
56. Use according to any one of clauses 52 to 55 wherein said measurement takes place over more than 1, 2, 3, 4, 5, 6, or 12 months.
57. A method of monitoring a stent comprising:
   transmitting a wireless electrical signal from a location outside the body to a location inside the body;
   receiving the signal at a sensor positioned on a stent located inside the body;
   powering the sensor using the received signal;
   sensing data at the sensor; and
   outputting the sensed data from the sensor to a receiving unit located outside of the body.
58. The method according to clause 57 wherein said stent is an assembly according to any one of clauses 1 to 51.
59. The method according to clause 57 or 58 wherein said receiving unit is a watch, writs band, cell phone or glasses.
60. The method according to any one of clauses 57 to 59 wherein said receiving unit is located within a subject's residence or office.
61. The method according to any one of clauses 57 to 60 wherein said sensed data is provided to a health care provider.
62. The method according to any one of clauses 57 to 61 wherein said sensed data is posted to one or more websites.
63. A non-transitory computer-readable storage medium whose stored contents configure a computing system to perform a method, the method comprising:
   identifying a subject, the identified subject having at least one wireless stent, each wireless stent having one or more wireless sensors;
   directing a wireless interrogation unit to collect sensor data from at least one of the respective one or more wireless sensors; and
   receiving the collected sensor data.
64. The non-transitory computer-readable storage medium of clause 63 whose stored contents configure a computing system to perform a method, the method further comprising:
   identifying a plurality of subjects, each identified subject having at least one wireless stent, each wireless stent having one or more wireless sensors;
   directing a wireless interrogation unit associated with each identified subject to collect sensor data from at least one of the respective one or more wireless sensors;
   receiving the collected sensor data; and
   aggregating the collected sensor data.
65. The non-transitory computer-readable storage medium of clause 63 whose stored contents configure a computing system to perform a method, the method further comprising:
   removing sensitive subject data from the collected sensor data; and
   parsing the aggregated data according to a type of sensor.
66. The non-transitory computer-readable storage medium of clause 63 whose stored contents configure a computing system to perform a method, wherein directing the wireless interrogation unit includes directing a control unit associated with the wireless interrogation unit.
67. The non-transitory computer readable storage medium according to any one of clauses 63 to 66, wherein said stent is an assembly according to any one of clauses 1 to 51.
68. The storage medium according to any one of clauses 63 to 67 wherein said collected sensor data is received on a watch, wrist band, cell phone or glasses.
69. The storage medium according to any one of clauses 63 to 68 wherein said collected sensor data is received within a subject's residence or office.
70. The storage medium according to any one of clauses 63 to 69 wherein said collected sensed data is provided to a health care provider.
71. The storage medium according to any one of clauses 63 to 70 wherein said sensed data is posted to one or more websites.
72. The method according to any one of clauses 57 to 62, or storage medium according to any one of clauses 63 to 71, wherein said data is analyzed.
73. The method or storage medium according to clause 72 wherein said data is plotted to enable visualization of change over time.
74. The method or storage medium according to clauses 72 or 73 wherein said data is plotted to provide a three-dimensional image.
75. A method for determining degradation of a stent, comprising the steps of a) providing to a body passageway of a subject an assembly comprising a stent and one or more sensors, and b) detecting a change in a sensor, and thus determining degradation of the stent.
76. The method according to clause 75 wherein said sensor is capable of detecting one or more physiological and/or locational parameters.
77. The method according to clause 75 or 76 wherein said sensor detects contact, fluid flow, pressure and/or temperature.
78. The method according to any one of clauses 75 to 77 wherein said sensor detects a location within the subject.
79. The method according to any one of clauses 75 to 78 wherein said assembly is an assembly according to clauses 1 to 51.
80. The method according to any one of clauses 75 to 79 wherein the step of detecting is a series of detections over time.
81. A method for imaging a stent, comprising detecting the changes in sensors in, on, and or within a stent over time, and wherein the stent comprises sensors at a density of greater than 1, 2, 3, 4, 5, 6, 7, 8, 9, 10 or 20 sensors per square centimeter.
82. A method for imaging a stent, comprising detecting changes in sensors in, on, and or within a stent over time, and wherein the stent comprises sensors at a density of greater than 1, 2, 3, 4, 5, 6, 7, 8, 9, 10 or 20 sensors per cubic centimeter.
83. The method according to clauses 81 or 82, wherein said sensor is one or more of a fluid pressure sensor, contact sensor, position sensor, accelerometer, pressure sensor, blood volume sensor, blood flow sensor, blood chemistry sensor, blood metabolic sensor, mechanical stress sensor, and temperature sensor.
84. The method according to any one of clauses 81 to 83 wherein said stent is an assembly according to any one of clauses 1 to 51.
85. A method for placing a stent within a subject, comprising a) implanting an assembly according to any one of clauses 1 to 51, and b) detecting placement of the stent by detecting a sensor.
86. The method according to clause 85 wherein the stent comprises two or more sections, and wherein detection of said two or more sections can be determined by analysis of one or more sensors.
87. The method according to clauses 85 or 86 wherein placement of the stent can be visualized by a two or three dimensional representation or image of the one or more sensors on said stent.
88. The method according to any one of clauses 85 to 87, wherein said method comprises two stents which are implanted to overlap with each other.
89. The method according to anyone of clauses 85 to 88 wherein said detecting placement of the stent allows determination of whether the stent is kinked or placed incorrectly.

## Claims

1. An assembly comprising a stent; and a sensor positioned on or within said stent, where the sensor is configured to measure the development of restenosis, and where measurements from the sensor are collected and stored in a memory located inside said stent.

2. The assembly according to claim 1 wherein said stent is comprised of two sections.

3. The assembly according to claim 2 wherein a sensor and a memory are positioned on each of the two sections.

4. The assembly according to any of claims 1-3 wherein said stent is a vascular stent.

5. The assembly according to any of claims 1-4 wherein the sensor is a fluid pressure sensor.

6. The assembly according to any of claims 1-4 wherein said sensor is a wireless sensor.

7. The assembly according to any of claims 1-4 wherein said sensor is a passive, wireless sensor.

8. The assembly according to any of claims 1-4 wherein said sensor is connected to a wireless microprocessor.

9. The assembly according to any preceding claim wherein said sensor has a unique sensor identification number.

10. A method of monitoring restenosis in a blood vessel of a body of a patient, the method comprising using the sensor of an assembly of any of claims 1-9 located in the blood vessel to obtain measurements of restenosis within the blood vessel, and storing said measurements in the memory.

11. The method of claim 10 wherein monitoring is performed over several weeks or several months.

12. The method of claim 10 or 11 wherein the patient has a reading device in their home which collates the data from the stent on a periodic basis, such as once per day or once per week.

13. The method of any one of claims 10-12 comprising (a) transmitting a wireless electrical signal from a location outside the body to a location inside the body; b) receiving the signal at the sensor positioned on the stent located inside the body, c) powering the sensor using the received signal, d) sensing data at the sensor, and e) outputting the sensed data from the sensor to a receiving unit located outside of the body.
